# EUROPEAN PATENT SPECIFICATION

(11) **EP 1 519 715 B1**
(45) Date of publication and mention of the grant of the patent: **22.02.2023**
(21) Application number: 03808363.0
(22) Date of filing: 23.05.2003
(51) Int. Cl.: A61K 9/14, A61K 9/20, A61K 31/216

(54) **NANOPARTICULATE FIBRATE FORMULATIONS**
NANOPARTIKULÄRE FIBRAT-FORMULIERUNGEN
PREPARATIONS DE FIBRATE NANOPARTICULAIRE

(30) Priority: 24.05.2002 US 383294 P; 21.02.2003 US 370277
(43) Date of publication of application: 06.04.2005
(73) Proprietor: Alkermes Pharma Ireland Limited, County Westmeath (IE); BGP Products Ireland Ltd., Dublin (IE)
(72) Inventor: RYDE, Tuula, Malvern, PA 19355 (US); GUSTOW, Evan, E., Villanova, PA 19085 (US); RUDDY, Stephen, B., Schwenksville, PA 19473 (US); JAIN, Rajeev c/o Elan Drug Delivery, King of Prussia, PA (US); PATEL, Rakesh, Bensalem, PA 19020 (US); WILKINS, Michael, John, Midleton, Co. Cork (IE)
(74) Representative: FRKelly
(86) International application number: PCT/US2003/014542
(87) International publication number: WO 2004/041250

(56) References cited:
- EP-A2- 0 499 299
- WO-A-01/21154
- US-A1- 2002 012 704
- US-A1- 2002 056 206
- US-B1- 6 177 103
- US-B1- 6 387 409
- US-B2- 6 368 620
- GARY G LIVERSIDGE; KENNETH C CUNDY: "Particle size reduction for improvement of oral bioavailability hydrophobic drugs; I. Absolute oral bioavailability of nanocrystalline danazol in beagle dogs", INTERNATIONAL JOURNAL OF PHARMACEUTICS, vol. 125, 1 January 1995 (1995-01-01), pages 91-97,
- Gary G Liversidge ET AL: "Drug particle size reduction for decreasing gastric irritancy and enhancing absorption of naproxen in rats", INTERNATIONAL JOURNAL OF PHARMACEUTICS, vol. 125, no. 2, 1 January 1995 (1995-01-01), pages 309-313, XP055501732, NL ISSN: 0378-5173, DOI: 10.1016/0378-5173(95)00148-C
- "First U.S. approval for Elan's Nanocrystal formulation", PHARMACEUTICAL ONLINE, 30 August 2000 (2000-08-30),
- "Sodium lauryl sulfate" In: "Handbook", 15 August 2005 (2005-08-15)

## Description

### FIELD OF THE INVENTION

The present invention relates to a nanoparticulate composition comprising fenofibrate or a salt thereof.

### BACKGROUND OF THE INVENTION

### A. Background Regarding Nanoparticulate Compositions

Nanoparticulate compositions, first described in U.S. Patent No. 5,145,684 ("the '684 patent"), are particles consisting of a poorly soluble therapeutic or diagnostic agent having adsorbed onto the surface thereof a non-crosslinked surface stabilizer. The '684 patent does not describe nanoparticulate compositions of a fibrate.

Methods of making nanoparticulate compositions are described in, for example, U.S. Patent Nos. 5,518,187 and 5,862,999, both for "Method of Grinding Pharmaceutical Substances;" U.S. Patent No. 5,718,388, for "Continuous Method of Grinding Pharmaceutical Substances;" and U.S. Patent No. 5,510,118 for "Process of Preparing Therapeutic Compositions Containing Nanoparticles."

Nanoparticulate compositions are also described, for example, in U.S. Patent Nos. 5,298,262 for "Use of Ionic Cloud Point Modifiers to Prevent Particle Aggregation During Sterilization;" 5,302,401 for "Method to Reduce Particle Size Growth During Lyophilization;" 5,318,767 for "X-Ray Contrast Compositions Useful in Medical Imaging;" 5,326,552 for "Novel Formulation For Nanoparticulate X-Ray Blood Pool Contrast Agents Using High Molecular Weight Non-ionic Surfactants;" 5,328,404 for "Method of X-Ray Imaging Using Iodinated Aromatic Propanedioates;" 5,336,507 for "Use of Charged Phospholipids to Reduce Nanoparticle Aggregation;" 5,340,564 for "Formulations Comprising Olin 10-G to Prevent Particle Aggregation and Increase Stability;" 5,346,702 for "Use of Non-Ionic Cloud Point Modifiers to Minimize Nanoparticulate Aggregation During Sterilization;" 5,349,957 for "Preparation and Magnetic Properties of Very Small Magnetic-Dextran Particles;" 5,352,459 for "Use of Purified Surface Modifiers to Prevent Particle Aggregation During Sterilization;" 5,399,363 and 5,494,683, both for "Surface Modified Anticancer Nanoparticles;" 5,401,492 for "Water Insoluble Non-Magnetic Manganese Particles as Magnetic Resonance Enhancement Agents;" 5,429,824 for "Use of Tyloxapol as a Nanoparticulate Stabilizer;" 5,447,710 for "Method for Making Nanoparticulate X-Ray Blood Pool Contrast Agents Using High Molecular Weight Non-ionic Surfactants;" 5,451,393 for "X-Ray Contrast Compositions Useful in Medical Imaging;" 5,466,440 for "Formulations of Oral Gastrointestinal Diagnostic X-Ray Contrast Agents in Combination with Pharmaceutically Acceptable Clays;" 5,470,583 for "Method of Preparing Nanoparticle Compositions Containing Charged Phospholipids to Reduce Aggregation;" 5,472,683 for "Nanoparticulate Diagnostic Mixed Carbamic Anhydrides as X-Ray Contrast Agents for Blood Pool and Lymphatic System Imaging;" 5,500,204 for "Nanoparticulate Diagnostic Dimers as X-Ray Contrast Agents for Blood Pool and Lymphatic System Imaging;" 5,518,738 for "Nanoparticulate NSAID Formulations;" 5,521,218 for "Nanoparticulate Iododipamide Derivatives for Use as X-Ray Contrast Agents;" 5,525,328 for "Nanoparticulate Diagnostic Diatrizoxy Ester X-Ray Contrast Agents for Blood Pool and Lymphatic System Imaging;" 5,543,133 for "Process of Preparing X-Ray Contrast Compositions Containing Nanoparticles;" 5,552,160 for "Surface Modified NSAID Nanoparticles;" 5,560,931 for "Formulations of Compounds as Nanoparticulate Dispersions in Digestible Oils or Fatty Acids;" 5,565,188 for "Polyalkylene Block Copolymers as Surface Modifiers for Nanoparticles;" 5,569,448 for "Sulfated Non-ionic Block Copolymer Surfactant as Stabilizer Coatings for Nanoparticle Compositions;" 5,571,536 for "Formulations of Compounds as Nanoparticulate Dispersions in Digestible Oils or Fatty Acids;" 5,573,749 for "Nanoparticulate Diagnostic Mixed Carboxylic Anydrides as X-Ray Contrast Agents for Blood Pool and Lymphatic System Imaging;" 5,573,750 for "Diagnostic Imaging X-Ray Contrast Agents;" 5,573,783 for "Redispersible Nanoparticulate Film Matrices With Protective Overcoats;" 5,580,579 for "Site-specific Adhesion Within the GI Tract Using Nanoparticles Stabilized by High Molecular Weight, Linear Poly(ethylene Oxide) Polymers;" 5,585,108 for "Formulations of Oral Gastrointestinal Therapeutic Agents in Combination with Pharmaceutically Acceptable Clays;" 5,587,143 for "Butylene Oxide-Ethylene Oxide Block Copolymers Surfactants as Stabilizer Coatings for Nanoparticulate Compositions;" 5,591,456 for "Milled Naproxen with Hydroxypropyl Cellulose as Dispersion Stabilizer;" 5,593,657 for "Novel Barium Salt Formulations Stabilized by Non-ionic and Anionic Stabilizers;" 5,622,938 for "Sugar Based Surfactant for Nanocrystals;" 5,628,981 for "Improved Formulations of Oral Gastrointestinal Diagnostic X-Ray Contrast Agents and Oral Gastrointestinal Therapeutic Agents;" 5,643,552 for "Nanoparticulate Diagnostic Mixed Carbonic Anhydrides as X-Ray Contrast Agents for Blood Pool and Lymphatic System Imaging;" 5,718,388 for "Continuous Method of Grinding Pharmaceutical Substances;" 5,718,919 for "Nanoparticles Containing the R(-)Enantiomer of Ibuprofen;" 5,747,001 for "Aerosols Containing Beclomethasone Nanoparticle Dispersions;" 5,834,025 for "Reduction of Intravenously Administered Nanoparticulate Formulation Induced Adverse Physiological Reactions;" 6,045,829 "Nanocrystalline Formulations of Human Immunodeficiency Virus (HIV) Protease Inhibitors Using Cellulosic Surface Stabilizers;" 6,068,858 for "Methods of Making Nanocrystalline Formulations of Human Immunodeficiency Virus (HIV) Protease Inhibitors Using Cellulosic Surface Stabilizers;" 6,153,225 for "Injectable Formulations of Nanoparticulate Naproxen;" 6,165,506 for "New Solid Dose Form of Nanoparticulate Naproxen;" 6,221,400 for "Methods of Treating Mammals Using Nanocrystalline Formulations of Human Immunodeficiency Virus (HIV) Protease Inhibitors;" 6,264,922 for "Nebulized Aerosols Containing Nanoparticle Dispersions;" 6,267,989 for "Methods for Preventing Crystal Growth and Particle Aggregation in Nanoparticle Compositions;" 6,270,806 for "Use of PEG-Derivatized Lipids as Surface Stabilizers for Nanoparticulate Compositions;" 6,316,029 for "Rapidly Disintegrating Solid Oral Dosage Form," 6,375,986 for "Solid Dose Nanoparticulate Compositions Comprising a Synergistic Combination of a Polymeric Surface Stabilizer and Dioctyl Sodium Sulfosuccinate;" 6,428,814 for "Bioadhesive Nanoparticulate Compositions Having Cationic Surface Stabilizers;" 6,431,478 for "Small Scale Mill;" and 6,432,381 for "Methods for Targeting Drug Delivery to the Upper and/or Lower Gastrointestinal Tract".. In addition, U.S. Patent Application No. 20020012675 A1, published on January 31, 2002, for "Controlled Release Nanoparticulate Compositions," describes nanoparticulate compositions.

Amorphous small particle compositions are described, for example, in U.S. Patent Nos. 4,783,484 for "Particulate Composition and Use Thereof as Antimicrobial Agent;" 4,826,689 for "Method for Making Uniformly Sized Particles from Water-Insoluble Organic Compounds;" 4,997,454 for "Method for Making Uniformly-Sized Particles From Insoluble Compounds;" 5,741,522 for "Ultrasmall, Non-aggregated Porous Particles of Uniform Size for Entrapping Gas Bubbles Within and Methods;" and 5,776,496, for "Ultrasmall Porous Particles for Enhancing Ultrasound Back Scatter." US2002012704 discloses a process for the preparation of small particles containing a poorly water soluble drug, which may be fenofibrate, comprising the steps of (a) mixing at high shear an admixture of a poorly water soluble drug and one or more than one surface active substance in an aqueous carrier in the absence of an organic solvent within a first temperature range at or above the melting point of the poorly water soluble drug to form a heated suspension containing the drug, then (b) homogenizing said heated suspension in a first pressure range and within said first temperature range to form a heated homogenate containing the drug, then (c) cooling said heated homogenate to a second temperature range below the melting temperature of the poorly water soluble drug to form a transiently stable cooled homogenate containing the drug, then (d) applying a particle stabilizing energetic process to said cooled homogenate within a second temperature range below the melting point of the drug and in a second pressure range to form a cooled dispersion of stabilized small particles containing the drug, and then (e) optionally drying the cooled dispersion to form dried small particles containing the poorly water soluble drug.

### B. Background Regarding Fenofibrate

The compositions of the invention comprise fenofibrate. Fenofibrate, also known as 2-[4-(4-chlorobenzoyl) phenoxy]-2-methyl-propanoic acid, 1-methylethyl ester, is a lipid regulating agent. The compound is insoluble in water. *See* The Physicians' Desk Reference, 56th Ed., pp. 513-516 (2002).

Fenofibrate is described in, for example, U.S. Patent Nos. 3,907,792 for "Phenoxy-Alkyl-Carboxylic Acid Derivatives and the Preparation Thereof;" 4,895,726 for "Novel Dosage Form of Fenofibrate;" 6,074,670 and 6,277,405, both for "Fenofibrate Pharmaceutical Composition Having High Bioavailability and Method for Preparing It." U.S. Patent No. 3,907,792 describes a class of phenoxy-alkyl carboxylic compounds which encompasses fenofibrate. U.S. Patent No. 4,895,726 describes a gelatin capsule therapeutic composition, useful in the oral treatment of hyerlipidemia and hypercholesterolemia, containing micronized fenofibrate. U.S. Patent No. 6,074,670 refers to immediate-release fenofibrate compositions comprising micronized fenofibrate and at least one inert hydrosoluble carrier. U.S. Patent No. 4,739,101 describes a process for making fenofibrate. U.S. Patent No. 6,277,405 is directed to micronized fenofibrate compositions having a specified dissolution profile. In addition, International Publication No. WO 02/24193 for "Stabilised Fibrate Microparticles," published on March 28, 2002, describes a microparticulate fenofibrate composition comprising a phospholipid. Finally, International Publication No. WO 02/067901 for "Fibrate-Statin Combinations with Reduced Fed-Fasted Effects," published on September 6, 2002, describes a microparticulate fenofibrate composition comprising a phospholipid and a hydroxymethylglutaryl coenzyme A (HMG-CoA) reductase inhibitor or statin.

WO 01/80828 for "Improved Water-Insoluble Drug Particle Process," and International Publication No. WO 02/24193 for "Stabilised Fibrate Microparticles," describe a process for making small particle compositions of poorly water soluble drugs. The process requires preparing an admixture of a drug and one or more surface active agents, followed by heating the drug admixture to at or above the melting point of the poorly water soluble drug. The heated suspension is then homogenized. The use of such a heating process is undesirable, as heating a drug to its melting point destroys the crystalline structure of the drug. Upon cooling, a drug may be amorphous or recrystallize in a different isoform, thereby producing a composition which is physically and structurally different from that desired. Such a "different" composition may have different pharmacological properties. This is significant as U.S. Food and Drug Administration (USFDA) approval of a drug substance requires that the drug substance be stable and produced in a repeatable process.

WO 03/013474 for "Nanoparticulate Formulations of Fenofibrate," published on February 20, 2003, describes fibrate compositions comprising vitamin E TGPS (polyethylene glycol (PEG) derivatized vitamin E). The fibrate compositions of this reference comprise particles of fibrate and vitamin E TPGS having a mean diameter from about 100 nm to about 900 nm (page 8, lines 12-15, of WO 03/013474), a D₅₀ of 350 - 750 nm, and a D₉₉ of 500 to 900 nm (page 9, lines 11-13, of WO 03/013474) (50% of the particles of a composition fall below a "D₅₀", and 99% of the particles of a composition fall below a D₉₉). The reference does *not* teach that the described compositions show minimal or no variability when administered in fed as compared to fasted conditions.

A variety of clinical studies have demonstrated that elevated levels of total cholesterol (total-C), low density lipoprotein cholesterol (LDL-C), and apolipoprotein B (apo B), an LDL membrane complex, are associated with human atherosclerosis. Similarly, decreased levels of high density lipoprotein cholesterol (HDL-C) and its transport complex, apolipoprotein A (apo A2 and apo AII), are associated with the development of atherosclerosis. Epidemiologic investigations have established that cardiovascular morbidity and mortality vary directly with the level of total-C, LDL-C, and triglycerides, and inversely with the level of HDL-C.

Fenofibric acid, the active metabolite of fenofibrate, produces reductions in total cholesterol, LDL cholesterol, apo-lipoprotein B, total triglycerides, and triglyceride rich lipoprotein (VLDL) in treated patients. In addition, treatment with fenofibrate results in increases in high density lipoprotein (HDL) and apolipoprotein apoAI and apoAII. *See* The Physicians' Desk Reference, 56th Ed., pp. 513-516 (2002).

Because fibrates, including fenofibrate, are so insoluble in water, significant bioavailability can be problematic. In addition, conventional fibrate, including fenofibrate, formulations exhibit dramatically different effects depending upon the fed or fasted state of the patient. Finally, conventional fibrate, including fenofibrate, formulations require relatively large doses to achieve the desired therapeutic effects. There is a need in the art for nanoparticulate fibrate formulations which overcome these and other problems associated with prior conventional microcrystalline fibrate formulations. The present invention satisfies these needs.

### SUMMARY OF THE INVENTION

The scope of the invention is defined by the claims.

The present invention relates to a fenofibrate composition for oral administration comprising:
(a) particles of fenofibrate or a salt thereof,
   wherein at least 99% of the fibrate particles by weight have a particle size of less than 500nm or at least 50% of the fibrate particles by weight have a particle size of less than 350nm; and
(b) associated with the surface thereof, a surface stabilizer composition comprising hypromellose, docusate sodium, and sodium lauryl sulfate,
   wherein the composition does not comprise PEG-derivatized vitamin E, and
   wherein the composition does not produce significantly different absorption levels when administered under fed as compared to fasting conditions
   wherein administration of the composition to a subject in a fasted state is bioequivalent to administration of the composition to a subject in a fed state, wherein "bioequivalency" is established by:
      (a) a 90% Confidence Interval (CI) of between 0.80 and 1.25 for both Cmax and AUC under USFDA regulatory guidelines or
      (b) a 90% Confidence Interval (CI) for AUC of between 0.80 and 1.25 and a 90% CI for Cmax of between 0.70 to 1.43 under the European EMEA regulatory guidelines.

A preferred dosage form of the invention is a solid dosage form, although any pharmaceutically acceptable dosage form can be utilized.

Another aspect of the disclosure is directed to a nanoparticulate fenofibrate composition having improved pharmacokinetic profiles as compared to conventional microcrystalline fibrate formulations, such as Tₘₐₓ, Cₘₐₓ, and AUC.

Another embodiment of the invention is directed to the nanoparticulate fenofibrate composition for use in treating dyslipidemia, hyperlipidemia, hypercholesterolemia, cardiovascular disorders, or related conditions.

Other embodiments of the disclosure include, but are not limited to, nanoparticulate fenofibrate formulations which, as compared to conventional non-nanoparticulate formulations of a fibrate, particularly a fenofibrate such as TRICOR^{®} (160 mg tablet or 200 mg capsule microcrystalline fenofibrate formulations), have one or more of the following properties: (1) smaller tablet or other solid dosage form size; (2) smaller doses of drug required to obtain the same pharmacological effect; (3) increased bioavailability; (4) substantially similar pharmacokinetic profiles of the nanoparticulate fenofibrate compositions when administered in the fed versus the fasted state; (5) an increased rate of dissolution for the nanoparticulate fenofibrate compositions; and (6) bioadhesive fenofibrate compositions.

This disclosure further relates to a (non-claimed) method of making a nanoparticulate fenofibrate composition according to the invention. Such a method comprises contacting fenofibrate and at least one surface stabilizer for a time and under conditions sufficient to provide a nanoparticulate fenofibrate composition, and preferably a fenofibrate composition. The one or more surface stabilizers can be contacted with the fenofibrate either before, during, or after size reduction of the fenofibrate.

The present invention is also directed to the nanoparticulate fenofibrate compositions of the invention for use in the treatment of conditions such as hypercholesterolemia, hypertriglyceridemia, coronary heart disease, and peripheral vascular disease (including symptomatic carotid artery disease). Although not claimed, it is envisaged that the compositions of the invention can be used as adjunctive therapy to diet for the reduction of LDL-C, total-C, triglycerides, and Apo B in adult patients with primary hypercholesterolemia or mixed dyslipidemia (Fredrickson Types IIa and IIb). It is also envisaged but not claimed that the compositions can also be used as adjunctive therapy to diet for treatment of adult patients with hypertriglyceridemia (Fredrickson Types IV and V hyperlipidemia). Markedly elevated levels of serum tryglycerides (*e.g*., > 2000 mg/dL) may increase the risk of developing pancreatitis. Such methods comprise administering to a subject a therapeutically effective amount of a nanoparticulate fenofibrate, composition according to the invention.

Both the foregoing general description and the following detailed description are exemplary and explanatory and are intended to provide further explanation of the invention as claimed. Other objects, advantages, and novel features will be readily apparent to those skilled in the art from the following detailed description of the invention.

### BRIEF DESCRIPTION OF THE DRAWINGS

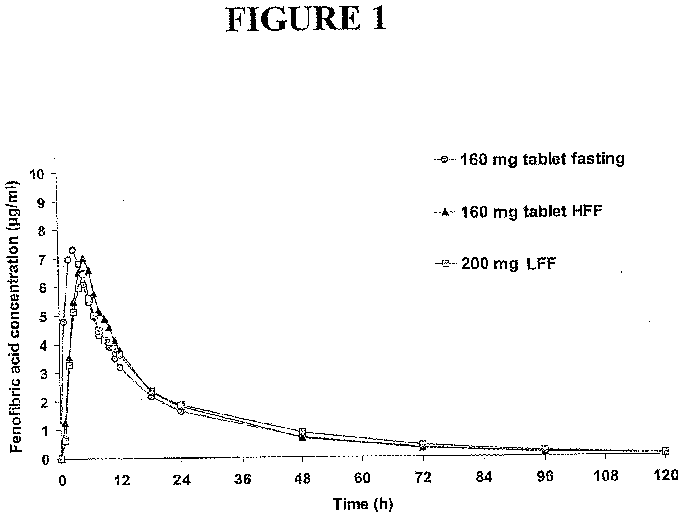

- Figure 1:: Shows the fenofibric acid concentration (µg/ml) over a period of 120 minutes for a single dose of: (a) a 160 mg nanoparticulate fenofibrate tablet administered to a fasting subject; (b) a 160 mg nanoparticulate fenofibrate tablet administered to a high fat fed subject; and (c) a 200 mg microcrystalline (TRICOR^{®}; Abbott Laboratories, Abbott Park, IL) capsule administered to a low fat fed subject; and

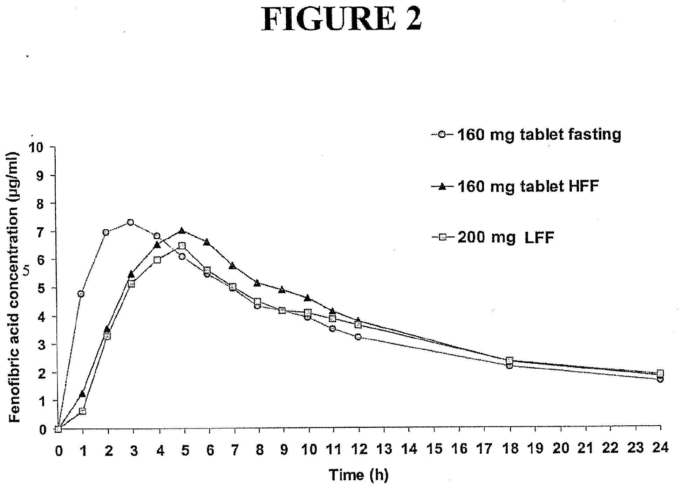

- Figure 2:: Shows the fenofibric acid concentration (µg/ml) over a period of 24 hours for a single dose of: (a) a 160 mg nanoparticulate fenofibrate tablet administered to a fasting subject; (b) a 160 mg nanoparticulate fenofibrate tablet administered to a high fat fed subject; and (c) a 200 mg microcrystalline (TRICOR^{®}) capsule administered to a low fat fed subject.

### DETAILED DESCRIPTION OF THE INVENTION

The present invention is directed to a fenofibrate composition for oral administration comprising:
(a) particles of fenofibrate or a salt thereof, wherein at least 99% of the fibrate particles by weight have a particle size of less than 500nm or at least 50% of the fibrate particles by weight have a particle size of less than 350nm; and
(b) associated with the surface thereof, a surface stabilizer composition comprising hypromellose, docusate sodium, and sodium lauryl sulfate, wherein the composition does not comprise PEG-derivatized vitamin E, and
   wherein the composition does not produce significantly different absorption levels when administered under fed as compared to fasting conditions
   wherein administration of the composition to a subject in a fasted state is bioequivalent to administration of the composition to a subject in a fed state, wherein "bioequivalency" is established by:
      (a) a 90% Confidence Interval (CI) of between 0.80 and 1.25 for both Cmax and AUC under USFDA regulatory guidelines or
      (b) a 90% Confidence Interval (CI) for AUC of between 0.80 and 1.25 and a 90% CI for Cmax of between 0.70 to 1.43 under the European EMEA regulatory guidelines.

As taught in the '684 patent, and as exemplified in the examples below, not every combination of surface stabilizer and active agent will result in a stable nanoparticulate composition. It was surprisingly discovered that stable, nanoparticulate fenofibrate formulations can be made.

Advantages of the nanoparticulate fenofibrate formulations of the invention as compared to conventional non-nanoparticulate formulations of a fibrate, particularly a fenofibrate such as TRICOR^{®} (tablet or capsule microcrystalline fenofibrate formulations), include, but are not limited to: (1) smaller tablet or other solid dosage form size; (2) smaller doses of drug required to obtain the same pharmacological effect; (3) increased bioavailability; (4) substantially similar pharmacokinetic profiles of the nanoparticulate fenofibrate compositions when administered in the fed versus the fasted state; (5) improved pharmacokinetic profiles; (6) bioequivalency of the nanoparticulate fenofibrate compositions when administered in the fed versus the fasted state; (7) an increased rate of dissolution for the nanoparticulate fenofibrate compositions; (8) bioadhesive fenofibrate compositions; and (9) the nanoparticulate fenofibrate compositions can be used in conjunction with other active agents useful in treating dyslipidemia, hyperlipidemia, hypercholesterolemia, cardiovascular disorders, or related conditions.

The present invention also includes nanoparticulate fenofibrate compositions together with one or more non-toxic physiologically acceptable carriers, adjuvants, or vehicles, collectively referred to as carriers. The compositions are formulated for oral administration.

A preferred dosage form of the invention is a solid dosage form, although any pharmaceutically acceptable dosage form can be utilized. Exemplary solid dosage forms include, but are not limited to, tablets, capsules, sachets, lozenges, powders, pills, or granules, and the solid dosage form can be, for example, a fast melt dosage form, controlled release dosage form, lyophilized dosage form, delayed release dosage form, extended release dosage form, pulsatile release dosage form, mixed immediate release and controlled release dosage form, or a combination thereof. A solid dose tablet formulation is preferred.

The present invention is described herein using several definitions, as set forth below and throughout the application.

As used herein, "about" will be understood by persons of ordinary skill in the art and will vary to some extent on the context in which it is used. If there are uses of the term which are not clear to persons of ordinary skill in the art given the context in which it is used, "about" will mean up to plus or minus 10% of the particular term.

As used herein with reference to stable fenofibrate particles, "stable" includes, but is not limited to, one or more of the following parameters: (1) that the fibrate particles do not appreciably flocculate or agglomerate due to interparticle attractive forces, or otherwise significantly increase in particle size over time; (2) that the physical structure of the fenofibrate particles is not altered over time, such as by conversion from an amorphous phase to crystalline phase; (3) that the fenofibrate particles are chemically stable; and/or (4) where the fibrate has not been subject to a heating step at or above the melting point of the fibrate in the preparation of the nanoparticles of the disclosure .

### A. Preferred Characteristics of the Fibrate Compositions of the Invention

### 1. Increased Bioavailability

The fenofibrate formulations of the invention exhibit increased bioavailability, at the same dose of the same fibrate, and require smaller doses as compared to prior conventional fenofibrate formulations.

For example, as shown below in Example 6, administration of a 160 mg nanoparticulate fenofibrate tablet in a fasted state is not bioequivalent to administration of a 200 mg conventional microcrystalline fenofibrate capsule (TRICOR^{®}) in a fed state, pursuant to regulatory guidelines. Under U.S. FDA guidelines, two products or methods are bioequivalent if the 90% Confidence Intervals (CI) for AUC and Cₘₐₓ are between 0.80 to 1.25 (Tₘₐₓ measurements are not relevant to bioequivalence for regulatory purposes). To show bioequivalency between two compounds or administration conditions pursuant to Europe's EMEA guidelines, the 90% CI for AUC must be between 0.80 to 1.25 and the 90% CI for Cₘₐₓ must between 0.70 to 1.43.

The non-bioequivalence is significant because it means that the nanoparticulate fenofibrate dosage form exhibits significantly greater drug absorption. For the nanoparticulate fenofibrate dosage form to be bioequivalent to the conventional microcrystalline fenofibrate dosage form (e.g., TRICOR^{®}), the nanoparticulate fenofibrate dosage form would have to contain significantly less drug. Thus, the nanoparticulate fenofibrate dosage form significantly increases the bioavailability of the drug.

Moreover, as shown below in Example 6, administration of a 160 mg nanoparticulate fenofibrate tablet in a fed state is bioequivalent to administration of a 200 mg conventional microcrystalline fenofibrate capsule (TRICOR^{®}) in a fed state. Thus, the nanoparticulate fenofibrate dosage form requires less drug to obtain the same pharmacological effect observed with the conventional microcrystalline fenofibrate dosage form (e.g., TRICOR^{®}). Therefore, the nanoparticulate fenofibrate dosage form has an increased bioavailability as compared to the conventional microcrystalline fenofibrate dosage form (e.g., TRICOR^{®}).

Greater bioavailability of the fibrate compositions of the disclosure can enable a smaller solid dosage size. This is particularly significant for patient populations such as the elderly, juvenile, and infant. In one embodiment of the disclosure , disclosed is a stable solid dose fenofibrate composition comprising: (a) a therapeutically effective dosage of 145 mg of particles of fenofibrate or a salt thereof; and (b) associated with the surface thereof at least one surface stabilizer.

### 2. Improved Pharmacokinetic Profiles

The disclosure also provides fenofibrate compositions having a desirable pharmacokinetic profile when administered to mammalian subjects. The desirable pharmacokinetic profile of the fenofibrate compositions comprise the parameters: (1) that the Tₘₐₓ of a fenofibrate when assayed in the plasma of the mammalian subject, is less than about 6 to about 8 hours. Preferably, the Tₘₐₓ parameter of the pharmacokinetic profile is less than about 6 hours, less than about 5 hours, less than about 4 hours, less than about 3 hours, less than about 2 hours, less than about 1 hour, or less than about 30 minutes after administration. The desirable pharmacokinetic profile, as used herein, is the pharmacokinetic profile measured after the initial dose of a fibrate, preferably fenofibrate. The compositions can be formulated in any way as described below and as known to those of skill in the art.

Current marketed formulations of fenofibrate include tablets, *i.e*., TRICOR^{®} tablets marketed by Abbott Laboratories. According to the description of TRICOR^{®}, the pharmacokinetic profile of the tablets contain parameters such that the median Tₘₐₓ is 6-8 hours (Physicians Desk Reference, 56th Ed., 2002). Because the compound is virtually insoluble in water, the absolute bioavailability of TRICOR^{®} cannot be determined (Physicians Desk Reference, 56th Ed., 2002). The compositions of the disclosure improve upon at least the Tₘₐₓ parameter of the pharmacokinetic profile of a fibrate, preferably fenofibrate.

A preferred fibrate formulation, preferably a fenofibrate formulation, of the disclosure exhibits in comparative pharmacokinetic testing with a standard commercial formulation of the same fibrate, *e.g.*, TRICOR^{®} tablets from Abbott Laboratories for fenofibrate, a Tₘₐₓ not greater than about 90%, not greater than about 80%, not greater than about 70%, not greater than about 60%, not greater than about 50%, not greater than about 30%, or not greater than about 25% of the Tₘₐₓ exhibited by a standard commercial fibrate formulation, *e.g.*, TRICOR^{®} tablets for fenofibrate.

Any formulation giving the desired pharmacokinetic profile is suitable for administration according to the present uses. Exemplary types of formulations giving such profiles are liquid dispersions, gels, aerosols, ointments, creams, solid dose forms, etc. of a nanoparticulate fibrate, preferably nanoparticulate fenofibrate.

In a preferred embodiment of the disclosure, a fenofibrate composition of the disclosure comprises fenofibrate or a salt thereof, which when administered to a human as a dose of about 160 mg presents an AUC of about 139 µg/mL.h.

In yet another preferred embodiment of the disclosure , a fenofibrate composition of the disclosure comprises fenofibrate and has a Cₘₐₓ under fasted conditions which is greater than the Cₘₐₓ under high fat fed (HFF) conditions, when administered to a human.

### 3. The Pharmacokinetic Profiles of the Fibrate Compositions of the Invention are not Affected by the Fed or Fasted State of the Subject Ingesting the Compositions

The disclosure encompasses a fenofibrate composition wherein the pharmacokinetic profile of the fibrate is not substantially affected by the fed or fasted state of a subject ingesting the composition, when administered to a human. This means that there is no substantial difference in the quantity of drug absorbed or the rate of drug absorption when the nanoparticulate fenofibrate compositions are administered in the fed versus the fasted state.

For conventional fenofibrate formulations, *i.e.,* TRICOR^{®}, the absorption of fenofibrate is increased by approximately 35% when administered with food. This significant difference in absorption observed with conventional fenofibrate formulations is undesirable. The fenofibrate formulations of the disclosure overcome this problem, as the fibrate formulations reduce or preferably substantially eliminate significantly different absorption levels when administered under fed as compared to fasting conditions when administered to a human.

In a preferred embodiment of the disclosure , a fenofibrate composition of the disclosure comprises about 145 mg of fenofibrate and exhibits minimal or no food effect when administered to a human. In another preferred embodiment of the disclosure , a fenofibrate composition of the disclosure comprises about 48 mg of fenofibrate and exhibits minimal or no food effect when administered to a human.

As shown in Example 6, the pharmacokinetic parameters of the fenofibrate compositions of the invention are the same when the composition is administered in the fed and fasted states when administered to a human. Specifically, there was no substantial difference in the rate or quantity of drug absorption when the fenofibrate composition was administered in the fed versus the fasted state. Thus, the fibrate compositions, and preferably fenofibrate compositions, of the invention substantially eliminate the effect of food on the pharmacokinetics of the fibrate when administered to a human.

Benefits of a dosage form which substantially eliminates the effect of food include an increase in subject convenience, thereby increasing subject compliance, as the subject does not need to ensure that they are taking a dose either with or without food. This is significant, as with poor subject compliance an increase in the medical condition for which the drug is being prescribed may be observed, *i.e*., cardiovascular problems for poor subject compliance with a fibrate such as fenofibrate.

### 4. Bioequivalency of the Fibrate Compositions of the Invention When Administered in the Fed Versus the Fasted State

The invention also encompasses a fibrate, preferably a fenofibrate, composition in which administration of the composition to a subject in a fasted state is bioequivalent to administration of the composition to a subject in a fed state. "Bioequivalency" is established by a 90% Confidence Interval (CI) of between 0.80 and 1.25 for both Cₘₐₓ and AUC under USFDA regulatory guidelines, or a 90% CI for AUC of between 0.80 to 1.25 and a 90% CI for Cₘₐₓ of between 0.70 to 1.43 under the European EMEA regulatory guidelines.

The difference in absorption of the fenofibrate compositions of the disclosure , when administered in the fed versus the fasted state, preferably is less than about 35%, less than about 30%, less than about 25%, less than about 20%, less than about 15%, less than about 10%, less than about 5%, or less than about 3%.

As shown in Example 6, administration of a fenofibrate composition according to the invention in a fasted state was bioequivalent to administration of a fenofibrate composition according to the invention in a fed state, pursuant to regulatory guidelines. Under USFDA guidelines, two products or methods are bioequivalent if the 90% Confidence Intervals (CI) for Cₘₐₓ (peak concentration) and the AUC (area under the concentration/time curve) are between 0.80 to 1.25. For Europe, the test for bioequivalency is if two products or methods have a 90% CI for AUC of between 0.80 to 1.25 and a 90% CI for Cₘₐₓ of between 0.70 to 1.43. The fenofibrate compositions of the invention meet both the U.S. and European guidelines for bioequivalency for administration in the fed versus the fasted state.

### 5. Dissolution Profiles of the Fibrate Compositions of the Invention

The fenofibrate compositions of the invention have unexpectedly dramatic dissolution profiles. Rapid dissolution of an administered active agent is preferable, as faster dissolution generally leads to faster onset of action and greater bioavailability. To improve the dissolution profile and bioavailability of fibrates, and in particulate fenofibrate, it would be useful to increase the drug's dissolution so that it could attain a level close to 100%.

The fenofibrate compositions of the disclosure preferably have a dissolution profile in which within about 5 minutes at least about 20% of the composition is dissolved. In other embodiments of the disclosure, at least about 30% or about 40% of the fenofibrate composition is dissolved within about 5 minutes. In yet other embodiments of the disclosure, preferably at least about 40%, about 50%, about 60%, about 70%, or about 80% of the fenofibrate composition is dissolved within about 10 minutes. Finally, in another embodiment of the disclosure, preferably at least about 70%, about 80%, about 90%, or about 100% of the fenofibrate composition is dissolved within about 20 minutes.

Dissolution is preferably measured in a medium which is discriminating. Such a dissolution medium will produce two very different dissolution curves for two products having very different dissolution profiles in gastric juices; *i.e.,* the dissolution medium is predictive of *in vivo* dissolution of a composition. An exemplary dissolution medium is an aqueous medium containing the surfactant sodium lauryl sulfate at 0.025 M. Determination of the amount dissolved can be carried out by spectrophotometry. The rotating blade method (European Pharmacopoeia) can be used to measure dissolution.

### 6. Redispersibility Profiles of the Fibrate Compositions of the Invention

An additional feature of the fenofibrate compositions of the disclosure is that the compositions redisperse such that at least 99% of the fenofibrate particles by weight have a particle size of less than 500nm or at least 50% of the fenofibrate particles by weight have a particle size of less than 350nm. This is significant, as if upon administration the nanoparticulate fibrate compositions of the invention did not redisperse to a substantially nanoparticulate particle size, then the dosage form may lose the benefits afforded by formulating the fibrate into a nanoparticulate particle size.

This is because nanoparticulate active agent compositions benefit from the small particle size of the active agent; if the active agent does not redisperse into the small particle sizes upon administration, then "clumps" or agglomerated active agent particles are formed, owing to the extremely high surface free energy of the nanoparticulate system and the thermodynamic driving force to achieve an overall reduction in free energy. With the formation of such agglomerated particles, the bioavailability of the dosage form may fall well below that observed with the liquid dispersion form of the nanoparticulate active agent.

Moreover, the nanoparticulate fenofibrate compositions of the invention exhibit dramatic redispersion of the nanoparticulate fibrate particles upon administration to a mammal, such as a human or animal, as demonstrated by reconstitution/redispersion in a biorelevant aqueous media such that at least 99% of the fenofibrate particles by weight have a particle size of less than 500nm or at least 50% of the fenofibrate particles by weight have a particle size of less than 350nm. Such biorelevant aqueous media can be any aqueous media that exhibit the desired ionic strength and pH, which form the basis for the biorelevance of the media. The desired pH and ionic strength are those that are representative of physiological conditions found in the human body. Such biorelevant aqueous media can be, for example, aqueous electrolyte solutions or aqueous solutions of any salt, acid, or base, or a combination thereof, which exhibit the desired pH and ionic strength.

Biorelevant pH is well known in the art. For example, in the stomach, the pH ranges from slightly less than 2 (but typically greater than 1) up to 4 or 5. In the small intestine the pH can range from 4 to 6, and in the colon it can range from 6 to 8. Biorelevant ionic strength is also well known in the art. Fasted state gastric fluid has an ionic strength of about 0.1M while fasted state intestinal fluid has an ionic strength of about 0.14. *See e.g.,* Lindahl et al., "Characterization of Fluids from the Stomach and Proximal Jejunum in Men and Women," Pharm. Res., 14 (4): 497-502 (1997).

It is believed that the pH and ionic strength of the test solution is more critical than the specific chemical content. Accordingly, appropriate pH and ionic strength values can be obtained through numerous combinations of strong acids, strong bases, salts, single or multiple conjugate acid-base pairs (*i.e*., weak acids and corresponding salts of that acid), monoprotic and polyprotic electrolytes, *etc.*

Representative electrolyte solutions can be, but are not limited to, HCl solutions, ranging in concentration from about 0.001 to about 0.1 M, and NaCl solutions, ranging in concentration from about 0.001 to about 0.1 M, and mixtures thereof. For example, electrolyte solutions can be, but are not limited to, about 0.1 M HCl or less, about 0.01 M HCl or less, about 0.001 M HCl or less, about 0.1 M NaCl or less, about 0.01 M NaCl or less, about 0.001 M NaCl or less, and mixtures thereof. Of these electrolyte solutions, 0.01 M HCl and/or 0.1 M NaCl, are most representative of fasted human physiological conditions, owing to the pH and ionic strength conditions of the proximal gastrointestinal tract.

Electrolyte concentrations of 0.001 M HCl, 0.01 M HCl, and 0.1 M HCl correspond to pH 3, pH 2, and pH 1, respectively. Thus, a 0.01 M HCl solution simulates typical acidic conditions found in the stomach. A solution of 0.1 M NaCl provides a reasonable approximation of the ionic strength conditions found throughout the body, including the gastrointestinal fluids, although concentrations higher than 0.1 M may be employed to simulate fed conditions within the human GI tract.

Exemplary solutions of salts, acids, bases or combinations thereof, which exhibit the desired pH and ionic strength, include but are not limited to phosphoric acid/phosphate salts + sodium, potassium and calcium salts of chloride, acetic acid/acetate salts + sodium, potassium and calcium salts of chloride, carbonic acid/bicarbonate salts + sodium, potassium and calcium salts of chloride, and citric acid/citrate salts + sodium, potassium and calcium salts of chloride.

In other embodiments of the invention, the redispersed at least 99% fenofibrate particles of the invention have a particle size of less than 500nm or at least 50% of the fibrate particles by weight have a particle size of less than 350nm.

Redispersibility can be tested using any suitable means known in the art. *See e.g.,* the example sections of U.S. Patent No. 6,375,986 for "Solid Dose Nanoparticulate Compositions Comprising a Synergistic Combination of a Polymeric Surface Stabilizer and Dioctyl Sodium Sulfosuccinate."

### 7. Fibrate Compositions Used in Conjunction with Other Active Agents

The fenofibrate compositions of the invention can additionally comprise one or more compounds useful in treating dyslipidemia, hyperlipidemia, hypercholesterolemia, cardiovascular disorders, or related conditions, or the fenofibrate compositions can be administered in conjunction with such a compound. Examples of such compounds include, but are not limited to, statins or HMG CoA reductase inhibitors and antihypertensives. Examples of antihypertensives include, but are not limited to diuretics ("water pills"), beta blockers, alpha blockers, alpha-beta blockers, sympathetic nerve inhibitors, angiotensin converting enzyme (ACE) inhibitors, calcium channel blockers, angiotensin receptor blockers (formal medical name angiotensin-2-receptor antagonists, known as "sartans" for short).

Examples of statins or HMG CoA reductase inhibitors include, but are not limited to, lovastatin; pravastatin; simavastatin; velostatin; atorvastatin (Lipitor^{®}) and other 6-[2-(substituted-pyrrol-1-yl)alkyl]pyran-2-ones and derivatives, as disclosed in U.S. Patent No. 4,647,576); fluvastatin (Lescol^{®}); fluindostatin (Sandoz XU-62-320); pyrazole analogs of mevalonolactone derivatives, as disclosed in PCT application WO 86/03488; rivastatin and other pyridyldihydroxyheptenoic acids, as disclosed in European Patent 491226A; Searle=s SC-45355 (a 3-substituted pentanedioic acid derivative); dichloroacetate; imidazole analogs of mevalonolactone, as disclosed in PCT application WO 86/07054; 3-carboxy-2-hydroxy-propane-phosphonic acid derivatives, as disclosed in French Patent No. 2,596,393; 2,3-di-substituted pyrrole, furan, and thiophene derivatives, as disclosed in European Patent Application No. 0221025; naphthyl analogs of mevalonolactone, as disclosed in U.S. Patent No. 4,686,237; octahydronaphthalenes, such as those disclosed in U.S. Patent No. 4,499,289; keto analogs of mevinolin (lovastatin), as disclosed in European Patent Application No. 0,142,146 A2; phosphinic acid compounds; as well as other HMG CoA reductase inhibitors.

### B. Compositions

The invention provides compositions comprising fenofibrate, particles and a stabilizer composition consisting of hypromellose, docusate sodium, and sodium lauryl sulfate. The surface stabilizer composition is associated with the surface of the fenofibrate particles. Surface stabilizers especially useful herein associate with the surface of the nanoparticulate fenofibrate particles but do not chemically react with the fenofibrate particles or itself. Individually adsorbed molecules of the surface stabilizer are essentially free of intermolecular cross-linkages.

The present invention also includes fenofenofibrate compositions together with one or more non-toxic physiologically acceptable carriers, adjuvants, or vehicles, collectively referred to as carriers. The compositions can be formulated for parenteral injection (e.g., intravenous, intramuscular, or subcutaneous), oral administration in solid, liquid, or aerosol form, vaginal, nasal, rectal, ocular, local (powders, ointments or drops), buccal, intracisternal, intraperitoneal, or topical administration, and the like.

### 1. Fibrate Particles

As used herein the term "fibrate" means any of the fibric acid derivatives. Fenofibrate is the fibrate compound used in this invention, but other examples of fibrate compounds are bezafibrate, beclobrate, binifibrate, ciplofibrate, clinofibrate, clofibrate, clofibric acid, etofibrate, gemfibrozil, nicofibrate, pirifibrate, ronifibrate, simfibrate, theofibrate, *etc. See* U.S. Patent No. 6,384,062.

Generally, fibrates are used for conditions such as hypercholesterolemia, mixed lipidemia, hypertriglyceridemia, coronary heart disease, and peripheral vascular disease (including symptomatic carotid artery disease), and prevention of pancreatitis. Fenofibrate may also help prevent the development of pancreatitis (inflammation of the pancreas) caused by high levels of triglycerides in the blood. Fibrates are known to be useful in treating renal failure (U.S. Patent No. 4,250,191). Fibrates may also be used for other indications where lipid regulating agents are typically used.

As used herein the term "fenofibrate" is used to mean fenofibrate (2-[4-(4-chlorobenzoyl) phenoxy]-2-methyl-propanoic acid, 1-methylethyl ester) or a salt thereof.

Fenofibrate is well known in the art and is readily recognized by one of ordinary skill. It is used to lower triglyceride (fat-like substances) levels in the blood. Specifically, fenofibrate reduces elevated LDL-C, Total-C, triglycerides, and Apo-B and increases HDL-C. The drug has also been approved as adjunctive therapy for the treatment of hypertriglyceridemia, a disorder characterized by elevated levels of very low density lipoprotein (VLDL) in the plasma.

The mechanism of action of fenofibrate has not been clearly established in man. Fenofibric acid, the active metabolite of fenofibrate, lowers plasma triglycerides apparently by inhibiting triglyceride synthesis, resulting in a reduction of VLDL released into the circulation, and also by stimulating the catabolism of triglyceride-rich lipoprotein (*i.e.,* VLDL). Fenofibrate also reduces serum uric acid levels in hyperuricemic and normal individuals by increasing the urinary excretion of uric acid.

The absolute bioavailability of conventional microcrystalline fenofibrate cannot be determined as the compound is virtually insoluble in aqueous media suitable for injection. However, fenofibrate is well absorbed from the gastrointestinal tract. Following oral administration in healthy volunteers, approximately 60% of a single dose of conventional radiolabelled fenofibrate (*i.e*., TRICOR^{®}) appeared in urine, primarily as fenofibric acid and its glucuronate conjugate, and 25% was excreted in the feces. *See* http://www.rxlist.com/cgi/generic3/fenofibrate_cp.htm

Following oral administration, fenofibrate is rapidly hydrolyzed by esterases to the active metabolite, fenofibric acid; no unchanged fenofibrate is detected in plasma. Fenofibric acid is primarily conjugated with glucuronic acid and then excreted in urine. A small amount of fenofibric acid is reduced at the carbonyl moiety to a benzhydrol metabolite which is, in turn, conjugated with glucuronic acid and excreted in urine. *Id.*

### 2. Surface Stabilizers

The choice of a surface stabilizer for a fibrate is non-trivial and required extensive experimentation to realize a desirable formulation. Accordingly, the present invention is directed to the surprising discovery that nanoparticulate fenofibrate compositions can be made.

The invention comprises a surface stabilizer composition comprising hypromellose, docusate sodium, and sodium lauryl sulfate. Combinations of more than one surface stabilizer can be used in the invention. Useful surface stabilizers which can also be employed in the invention include, but are not limited to, known organic and inorganic pharmaceutical excipients. Such excipients include various polymers, low molecular weight oligomers, natural products, and surfactants. Surface stabilizers include nonionic, anionic, cationic, ionic, and zwitterionic surfactants.

If desirable, the nanoparticulate fenofibrate compositions of the invention can be formulated to be phospholipid-free.

The surface stabilizers are commercially available and/or can be prepared by techniques known in the art. Most of these surface stabilizers are known pharmaceutical excipients and are described in detail in the *Handbook of Pharmaceutical Excipients,* published jointly by the American Pharmaceutical Association and The Pharmaceutical Society of Great Britain (The Pharmaceutical Press, 2000).

### 3. Other Pharmaceutical Excipients

Pharmaceutical compositions according to the disclosure may also comprise one or more binding agents, filling agents, lubricating agents, suspending agents, sweeteners, flavoring agents, preservatives, buffers, wetting agents, disintegrants, effervescent agents, and other excipients. Such excipients are known in the art.

Examples of filling agents are lactose monohydrate, lactose anhydrous, and various starches; examples of binding agents are various celluloses and cross-linked polyvinylpyrrolidone, microcrystalline cellulose, such as Avicel^{®} PH101 and Avicel^{®} PH102, microcrystalline cellulose, and silicified microcrystalline cellulose (ProSolv SMCC^{™}).

Suitable lubricants, including agents that act on the flowability of the powder to be compressed, are colloidal silicon dioxide, such as Aerosil^{®} 200, talc, stearic acid, magnesium stearate, calcium stearate, and silica gel.

Examples of sweeteners are any natural or artificial sweetener, such as sucrose, xylitol, sodium saccharin, cyclamate, aspartame, and acsulfame. Examples of flavoring agents are Magnasweet^{®} (trademark of MAFCO), bubble gum flavor, and fruit flavors, and the like.

Examples of preservatives are potassium sorbate, methylparaben, propylparaben, benzoic acid and its salts, other esters of parahydroxybenzoic acid such as butylparaben, alcohols such as ethyl or benzyl alcohol, phenolic compounds such as phenol, or quarternary compounds such as benzalkonium chloride.

Suitable diluents include pharmaceutically acceptable inert fillers, such as microcrystalline cellulose, lactose, dibasic calcium phosphate, saccharides, and/or mixtures of any of the foregoing. Examples of diluents include microcrystalline cellulose, such as Avicel^{®} PH101 and Avicel^{®} PH102; lactose such as lactose monohydrate, lactose anhydrous, and Pharmatose^{®} DCL21; dibasic calcium phosphate such as Emcompress^{®}; mannitol; starch; sorbitol; sucrose; and glucose.

Suitable disintegrants include lightly crosslinked polyvinyl pyrrolidone, corn starch, potato starch, maize starch, and modified starches, croscarmellose sodium, crosspovidone, sodium starch glycolate, and mixtures thereof.

Examples of effervescent agents are effervescent couples such as an organic acid and a carbonate or bicarbonate. Suitable organic acids include, for example, citric, tartaric, malic, fumaric, adipic, succinic, and alginic acids and anhydrides and acid salts. Suitable carbonates and bicarbonates include, for example, sodium carbonate, sodium bicarbonate, potassium carbonate, potassium bicarbonate, magnesium carbonate, sodium glycine carbonate, L-lysine carbonate, and arginine carbonate. Alternatively, only the sodium bicarbonate component of the effervescent couple may be present.

### 4. Nanoparticulate Fibrate Particle Size

The compositions of the invention contain nanoparticulate fibrate particles, preferably nanoparticulate fenofibrate particles, wherein at least 99% of the fenofibrate particles by weight have a particle size of less than 500nm or at least 50% of the fenofibrate particles by weight have a particle size of less than 350nm, as measured by light-scattering methods, microscopy, or other appropriate methods.

In one embodiment of the invention, at least 99% of the fibrate particles ("D₉₉") have a particle size less than about 500 nm, less than about 450 nm, less than about 400 nm, less than about 350 nm, less than about 300 nm, less than about 250 nm, less than about 200 nm, less than about 150 nm, or less than about 100 nm. In another embodiment of the invention, at least 50% of the fibrate particles ("D₅₀") have a particle size less than about 350 nm, less than about 300 nm, less than about 250 nm, less than about 200 nm, less than about 150 nm, less than about 100 nm, or less than about 75 nm. In yet another embodiment of the invention, the mean particle size of the fibrate composition is less than about 100 nm, less than about 75 nm, or less than about 50 nm.

In the present invention, the value for D50 of a nanoparticulate fenofibrate composition is the particle size below which 50% of the fibrate particles fall, by weight. Similarly, D90 is the particle size below which 90% of the fenofibrate particles fall, by weight.

### 5. Concentration of the Fibrate and Surface Stabilizers

The relative amounts of a fenofibrate and one or more surface stabilizers can vary widely. The optimal amount of the individual components can depend, for example, upon the particular fibrate selected, the hydrophilic lipophilic balance (HLB), melting point, and the surface tension of water solutions of the stabilizer, *etc.*

The concentration of the fenofibrate can vary from about 99.5% to about 0.001%, from about 95% to about 0.1%, or from about 90% to about 0.5%, by weight, based on the total combined weight of the fenofibrate and at least one surface stabilizer, not including other excipients.

The concentration of the at least one surface stabilizer can vary from about 0.5% to about 99.999%, from about 5.0% to about 99.9%, or from about 10% to about 99.5%, by weight, based on the total combined dry weight of the fenofibrate and the surface stabilizer composition, not including other excipients.

### 6. Exemplary Nanoparticulate Fenofibrate Tablet Formulations

Several exemplary fenofibrate tablet formulations of the invention are given below. These examples are not intended to limit the claims in any respect, but rather provide exemplary tablet formulations of fenofibrate of the invention which can be utilized in the uses of the invention. Such exemplary tablets can also comprise a coating agent.

| **Exemplary Nanoparticulate** | |
|---|---|
| **Fenofibrate Tablet Formulation #1** | |
| **Component** | **g/Kg** |
| Fenofibrate | about 50 to about 500 |
| Hypromellose, USP | about 10 to about 70 |
| Docusate Sodium, USP | about 1 to about 10 |
| Sucrose, NF | about 100 to about 500 |
| Sodium Lauryl Sulfate, NF | about 1 to about 40 |
| Lactose Monohydrate, NF | about 50 to about 400 |
| Silicified Microcrystalline Cellulose | about 50 to about 300 |
| Crospovidone, NF | about 20 to about 300 |
| Magnesium Stearate, NF | about 0.5 to about 5 |

| **Exemplary Nanoparticulate** | |
|---|---|
| **Fenofibrate Tablet Formulation #2** | |
| **Component** | **g/Kg** |
| Fenofibrate | about 100 to about 300 |
| Hypromellose, USP | about 30 to about 50 |
| Docusate Sodium, USP | about 0.5 to about 10 |
| Sucrose, NF | about 100 to about 300 |
| Sodium Lauryl Sulfate, NF | about 1 to about 30 |
| Lactose Monohydrate, NF | about 100 to about 300 |
| Silicified Microcrystalline Cellulose | about 50 to about 200 |
| Crospovidone, NF | about 50 to about 200 |
| Magnesium Stearate, NF | about 0.5 to about 5 |

| **Exemplary Nanoparticulate** | |
|---|---|
| **Fenofibrate Tablet Formulation #3** | |
| **Component** | **g/Kg** |
| Fenofibrate | about 200 to about 225 |
| Hypromellose, USP | about 42 to about 46 |
| Docusate Sodium, USP | about 2 to about 6 |
| Sucrose, NF | about 200 to about 225 |
| Sodium Lauryl Sulfate, NF | about 12 to about 18 |
| Lactose Monohydrate, NF | about 200 to about 205 |
| Silicified Microcrystalline Cellulose | about 130 to about 135 |
| Crospovidone, NF | about 112 to about 118 |
| Magnesium Stearate, NF | about 0.5 to about 3 |

| **Exemplary Nanoparticulate** | |
|---|---|
| **Fenofibrate Tablet Formulation #4** | |
| **Component** | **g/Kg** |
| Fenofibrate | about 119 to about 224 |
| Hypromellose, USP | about 42 to about 46 |
| Docusate Sodium, USP | about 2 to about 6 |
| Sucrose, NF | about 119 to about 224 |
| Sodium Lauryl Sulfate, NF | about 12 to about 18 |
| Lactose Monohydrate, NF | about 119 to about 224 |
| Silicified Microcrystalline Cellulose | about 129 to about 134 |
| Crospovidone, NF | about 112 to about 118 |
| Magnesium Stearate, NF | about 0.5 to about 3 |

### 7. Exemplary Embodiments of the Invention

The invention encompasses a fenofibrate composition for oral administration comprising:
(a) particles of fenofibrate or a salt thereof,
   wherein at least 99% of the fibrate particles by weight have a particle size of less than 500nm or at least 50% of the fibrate particles by weight have a particle size of less than 350nm; and
(b) associated with the surface thereof, a surface stabilizer composition comprising hypromellose, docusate sodium, and sodium lauryl sulfate,
   wherein the composition does not comprise PEG-derivatized vitamin E, and
   wherein the composition does not produce significantly different absorption levels when administered under fed as compared to fasting conditions
   wherein administration of the composition to a subject in a fasted state is bioequivalent to administration of the composition to a subject in a fed state, wherein "bioequivalency" is established by:
      (a) a 90% Confidence Interval (CI) of between 0.80 and 1.25 for both Cmax and AUC under USFDA regulatory guidelines or
      (b) a 90% Confidence Interval (CI) for AUC of between 0.80 and 1.25 and a 90% CI for Cmax of between 0.70 to 1.43 under the European EMEA regulatory guidelines

For any of the compositions described above, the fibrate is fenofibrate or a salt thereof.

### D. Methods of Making Nanoparticulate Fibrate Compositions

The nanoparticulate fenofibrate compositions can be made using, for example, milling, homogenization, or precipitation techniques. Exemplary (non-claimed) methods of making nanoparticulate compositions are described in the '684 patent. Methods of making nanoparticulate compositions are also described in U.S. Patent No. 5,518,187 for "Method of Grinding Pharmaceutical Substances;" U.S. Patent No. 5,718,388 for "Continuous Method of Grinding Pharmaceutical Substances;" U.S. Patent No. 5,862,999 for "Method of Grinding Pharmaceutical Substances;" U.S. Patent No. 5,665,331 for "Co-Microprecipitation of Nanoparticulate Pharmaceutical Agents with Crystal Growth Modifiers;" U.S. Patent No. 5,662,883 for "Co-Microprecipitation of Nanoparticulate Pharmaceutical Agents with Crystal Growth Modifiers;" U.S. Patent No. 5,560,932 for "Microprecipitation of Nanoparticulate Pharmaceutical Agents;" U.S. Patent No. 5,543,133 for "Process of Preparing X-Ray Contrast Compositions Containing Nanoparticles;" U.S. Patent No. 5,534,270 for "Method of Preparing Stable Drug Nanoparticles;" U.S. Patent No. 5,510,118 for "Process of Preparing Therapeutic Compositions Containing Nanoparticles;" and U.S. Patent No. 5,470,583 for "Method of Preparing Nanoparticle Compositions Containing Charged Phospholipids to Reduce Aggregation".

The resultant nanoparticulate fenofibrate compositions or dispersions can be utilized in solid or liquid dosage formulations, such as liquid dispersions, oral suspensions, gels, aerosols, ointments, creams, controlled release formulations, fast melt formulations, lyophilized formulations, tablets, capsules, delayed release formulations, extended release formulations, pulsatile release formulations, mixed immediate release and controlled release formulations, *etc.*

In one embodiment of the disclosure, if heat is utilized during the process of making the nanoparticulate composition, the temperature is kept below the melting point of the fibrate, preferably fenofibrate.

### 1. Milling to Obtain Nanoparticulate Fibrate Dispersions

Milling a fenofibrate to obtain a nanoparticulate dispersion comprises dispersing the fibrate particles in a liquid dispersion medium in which the fibrate is poorly soluble, followed by applying mechanical means in the presence of grinding media to reduce the particle size of the fibrate to the desired effective average particle size. The dispersion medium can be, for example, water, safflower oil, ethanol, t-butanol, glycerin, polyethylene glycol (PEG), hexane, or glycol. A preferred dispersion medium is water.

The fenofibrate particles can be reduced in size in the presence of at least one surface stabilizer. Alternatively, the fibrate particles can be contacted with one or more surface stabilizers after attrition. Other compounds, such as a diluent, can be added to the fibrate/surface stabilizer composition during the size reduction process. Dispersions can be manufactured continuously or in a batch mode.

In one embodiment of the disclosure, a mixture of a fibrate and one or more surface stabilizers is heated during the milling process. If a polymeric surface stabilizer is utilized, the temperature is raised to above the cloud point of the polymeric surface stabilizer but below the actual or depressed melting point of the fibrate. The utilization of heat may be important for scale up of the milling process, as it can aid in the solubilization of the one or more active agents.

### 2. Precipitation to Obtain Nanoparticulate Fibrate Compositions

Another (non-claimed) method of forming the desired nanoparticulate fenofibrate composition is by microprecipitation. This is a method of preparing stable dispersions of poorly soluble active agents in the presence of one or more surface stabilizers and one or more colloid stability enhancing surface active agents free of any trace toxic solvents or solubilized heavy metal impurities. Such a method comprises, for example: (1) dissolving a fibrate in a suitable solvent; (2) adding the formulation from step (1) to a solution comprising at least one surface stabilizer; and (3) precipitating the formulation from step (2) using an appropriate non-solvent. The method can be followed by removal of any formed salt, if present, by dialysis or diafiltration and concentration of the dispersion by conventional means.

### 3. Homogenization to Obtain Nanoparticulate Fibrate Compositions

Exemplary (non-claimed) homogenization methods of preparing active agent nanoparticulate compositions are described in U.S. Patent No. 5,510,118, for "Process of Preparing Therapeutic Compositions Containing Nanoparticles." Such a method comprises dispersing particles of a fenofibrate in a liquid dispersion medium, followed by subjecting the dispersion to homogenization to reduce the particle size of the fibrate to the desired effective average particle size. The fibrate particles can be reduced in size in the presence of at least one surface stabilizer. Alternatively, the fibrate particles can be contacted with one or more surface stabilizers either before or after attrition. Other compounds, such as a diluent, can be added to the fenofibrate/surface stabilizer composition either before, during, or after the size reduction process. Dispersions can be manufactured continuously or in a batch mode.

### 4. Exemplary Embodiments of the Invention

The disclosure encompasses a (not claimed) method of making a fibrate composition of the invention comprising contacting fibrate particles with a surface stabilizer composition for a time and under conditions sufficient to provide a fenofibrate composition for oral administration comprising:
(a) particles of fenofibrate or a salt thereof,
   wherein at least 99% of the fibrate particles by weight have a particle size of less than 500nm or at least 50% of the fibrate particles by weight have a particle size of less than 350nm; and
(b) associated with the surface thereof, a surface stabilizer composition comprising hypromellose, docusate sodium, and sodium lauryl sulfate,
   wherein the composition does not comprise PEG-derivatized vitamin E.
For such methods the contacting can comprise: (1) grinding, such as wet grinding, or (2) homogenizing; or (3) a process comprising (a) dissolving the fibrate particles in a solvent; (b) adding the resulting fibrate solution to a solution comprising at least one surface stabilizer; and (c) precipitating the solubilized fibrate having at least one surface stabilizer adsorbed on the surface thereof by the addition thereto of a non-solvent.

For any of the previous methods, the fibrate can be fenofibrate.

### D. Methods of Using the Fibrate Compositions of the Invention

The disclosure provides a (non-claimed) method of rapidly increasing the plasma levels of a fenofibrate in a subject. Such a method comprises orally administering to a subject an effective amount of a composition comprising a fibrate, preferably fenofibrate. The fibrate composition, when tested in fasting subjects in accordance with standard pharmacokinetic practice, produces a maximum blood plasma concentration profile in less than about 6 hours, less than about 5 hours, less than about 4 hours, less than about 3 hours, less than about 2 hours, less than about 1 hour, or less than about 30 minutes after the initial dose of the composition.

The compositions of the invention are useful in treating conditions such as hypercholesterolemia, hypertriglyceridemia, cardiovascular disorders, coronary heart disease, and peripheral vascular disease (including symptomatic carotid artery disease). Although not claimed, it is envisaged that the compositions of the invention can be used as adjunctive therapy to diet for the reduction of LDL-C, total-C, triglycerides, and Apo B in adult patients with primary hypercholesterolemia or mixed dyslipidemia (Fredrickson Types IIa and IIb). It is also envisaged but not claimed that the compositions can also be used as adjunctive therapy to diet for treatment of adult patients with hypertriglyceridemia (Fredrickson Types IV and V hyperlipidemia). Markedly elevated levels of serum tryglycerides (*e.g*., > 2000 mg/dL) may increase the risk of developing pancreatitis. Furthermore, although not claimed, it is envisaged that compositions of the invention can also be used for other indications where lipid regulating agents are typically used.

The fenofibrate compositions of the invention can be administered to a subject orally. As used herein, the term "subject" is used to mean an animal, preferably a mammal, including a human or non-human. The terms patient and subject may be used interchangeably.

The nanoparticulate fenofibrate compositions may also contain adjuvants such as preserving, wetting, emulsifying, and dispensing agents. Prevention of the growth of microorganisms can be ensured by various antibacterial and antifungal agents, such as parabens, chlorobutanol, phenol, sorbic acid, and the like. It may also be desirable to include isotonic agents, such as sugars, sodium chloride, and the like. Prolonged absorption of the injectable pharmaceutical form can be brought about by the use of agents delaying absorption, such as aluminum monostearate and gelatin.

Solid dosage forms for oral administration include, but are not limited to, capsules, tablets, pills, powders, and granules. In such solid dosage forms, the active agent may be admixed with at least one of the following: (a) one or more inert excipients (or carriers), such as sodium citrate or dicalcium phosphate; (b) fillers or extenders, such as starches, lactose, sucrose, glucose, mannitol, and silicic acid; (c) binders, such as carboxymethylcellulose, alignates, gelatin, polyvinylpyrrolidone, sucrose, and acacia; (d) humectants, such as glycerol; (e) disintegrating agents, such as agar-agar, calcium carbonate, potato or tapioca starch, alginic acid, certain complex silicates, and sodium carbonate; (f) solution retarders, such as paraffin; (g) absorption accelerators, such as quaternary ammonium compounds; (h) wetting agents, such as cetyl alcohol and glycerol monostearate; (i) adsorbents, such as kaolin and bentonite; and (j) lubricants, such as talc, calcium stearate, magnesium stearate, solid polyethylene glycols, sodium lauryl sulfate, or mixtures thereof. For capsules, tablets, and pills, the dosage forms may also comprise buffering agents.

Liquid dosage forms for oral administration include pharmaceutically acceptable emulsions, solutions, suspensions, syrups, and elixirs. In addition to the fibrate, the liquid dosage forms may comprise inert diluents commonly used in the art, such as water or other solvents, solubilizing agents, and emulsifiers. Exemplary emulsifiers are ethyl alcohol, isopropyl alcohol, ethyl carbonate, ethyl acetate, benzyl alcohol, benzyl benzoate, propyleneglycol, 1,3-butyleneglycol, dimethylformamide, oils, such as cottonseed oil, groundnut oil, corn germ oil, olive oil, castor oil, and sesame oil, glycerol, tetrahydrofurfuryl alcohol, polyethyleneglycols, fatty acid esters of sorbitan, or mixtures of these substances, and the like.

Besides such inert diluents, the composition can also include adjuvants, such as wetting agents, emulsifying and suspending agents, sweetening, flavoring, and perfuming agents.

"Therapeutically effective amount" as used herein with respect to a fibrate, preferably a fenofibrate, dosage shall mean that dosage that provides the specific pharmacological response for which the fibrate is administered in a significant number of subjects in need of such treatment. It is emphasized that "therapeutically effective amount," administered to a particular subject in a particular instance may not be effective for 100% of patients treated for a specific disease, and will not always be effective in treating the diseases described herein, even though such dosage is deemed a "therapeutically effective amount" by those skilled in the art. It is to be further understood that fibrate dosages are, in particular instances, measured as oral dosages, or with reference to drug levels as measured in blood.

One of ordinary skill will appreciate that effective amounts of a fibrate, such as fenofibrate, can be determined empirically and can be employed in pure form or, where such forms exist, in pharmaceutically acceptable salt, ester, or prodrug form. Actual dosage levels of a fibrate, such as fenofibrate, in the nanoparticulate compositions of the invention may be varied to obtain an amount of the fibrate that is effective to obtain a desired therapeutic response for a particular composition and method of administration. The selected dosage level therefore depends upon the desired therapeutic effect, the route of administration, the potency of the administered fibrate, the desired duration of treatment, and other factors.

Dosage unit compositions may contain such amounts of such submultiples thereof as may be used to make up the daily dose. It will be understood, however, that the specific dose level for any particular patient will depend upon a variety of factors: the type and degree of the cellular or physiological response to be achieved; activity of the specific agent or composition employed; the specific agents or composition employed; the age, body weight, general health, sex, and diet of the patient; the time of administration, route of administration, and rate of excretion of the agent; the duration of the treatment; drugs used in combination or coincidental with the specific agent; and like factors well known in the medical arts.

### 1. Exemplary Embodiments of the Invention

The invention encompasses a fenofibrate composition for oral administration comprising:
(a) particles of fenofibrate or a salt thereof,
   wherein at least 99% of the fibrate particles by weight have a particle size of less than 500nm or at least 50% of the fibrate particles by weight have a particle size of less than 350nm; and
(b) associated with the surface thereof, a surface stabilizer composition comprising hypromellose, docusate sodium, and sodium lauryl sulfate,
   wherein the composition does not comprise PEG-derivatized vitamin E, and
   wherein the composition does not produce significantly different absorption levels when administered under fed as compared to fasting conditions
   wherein administration of the composition to a subject in a fasted state is bioequivalent to administration of the composition to a subject in a fed state, wherein "bioequivalency" is established by:
      (a) a 90% Confidence Interval (CI) of between 0.80 and 1.25 for both Cmax and AUC under USFDA regulatory guidelines or
      (b) a 90% Confidence Interval (CI) for AUC of between 0.80 and 1.25 and a 90% CI for Cmax of between 0.70 to 1.43 under the European EMEA regulatory guidelines, for use in the treatment of a condition selected from the group consisting of hypercholesterolemia, hypertriglyceridemia, coronary heart disease, cardiovascular disorders, and peripheral vascular disease.

The following examples are given to illustrate the present invention.

Several of the formulations in the examples that follow were investigated using a light microscope. Here, "stable" nanoparticulate dispersions (uniform Brownian motion) were readily distinguishable from "aggregated" dispersions (relatively large, nonuniform particles without motion).

### Example 1

The purpose of this example was to prepare nanoparticulate dispersions of fenofibrate, and to test the prepared compositions for stability in water and in various simulated biological fluids.

Two formulations of fenofibrate were milled, as described in Table 1, by milling the components of the compositions under high energy milling conditions in a DYNO^{®}-Mill KDL (Willy A. Bachofen AG, Maschinenfabrik, Basle, Switzerland) for ninety minutes.

Formulation 1 comprised 5% (w/w) fenofibrate, 1% (w/w) hypromellose, and 0.05% (w/w) dioctyl sodium sulfosuccinate (DOSS), and Formulation 2 comprised 5% (w/w) fenofibrate, 1% (w/w) Pluronic^{®} S-630 (a random copolymer of vinyl acetate and vinyl pyrrolidone), and 0.05% (w/w) DOSS. The particle size of the resultant compositions was measured using a Horiba LA-910 Laser Scattering Particle Size Distribution Analyzer ((Horiba Instruments, Irvine, CA).

| **TABLE 1** | | | |
|---|---|---|---|
| **Nanoparticulate Fenofibrate Formulations Milled Under High Energy Conditions (Comparative Examples)** | | | |
| **Formulation** | **Drug** | **Surface Stabilizer** | **Particle Size** |
| 1 | 5% (w/w) | 1% hypromellose and 0.05% DOSS | Mean: 139 nm |
| | | | 90% < 266 nm |
| 2 | 5% (w/w) | 1% S630 and 0.05% DOSS | Mean: 233 nm |
| | | | 90% < 355 nm |

Next, the stability of the two formulations was tested in various simulated biological fluids (Table 2) and in water (Table 3) over an extended period of time. For tests in various simulated biological fluids, the composition was deemed stable if the particles remained in a dispersion format with no visible size increase or agglomeration after 30 min. incubation at 40°C. Testing in fluids representing electrolyte fluids is useful as such fluids are representative of physiological conditions found in the human body.

| **TABLE 2** | | | |
|---|---|---|---|
| **Stability Testing of Nanoparticulate Fenofibrate** | | | |
| **Formulations 1 and 2 in Simulated Biological Fluids** | | | |
| **Formulatio n** | **Electrolyte Test Media #1** | **Electrolyte Test Media #2** | **Electrolyte Test Media #3** |
| 1 | Slight Agglomeration | Acceptable | Acceptable |
| 2 | Heavy Agglomeration | Acceptable | Slight Agglomeration |

| **TABLE 3** | | | | |
|---|---|---|---|---|
| **Stability Testing of Nanoparticulate Fenofibrate** | | | | |
| **Formulations 1 and 2 in Water at 2-8°C** | | | | |
| **Formulation** | **3 Davs** | **1 Week** | **2 Weeks** | **7 Months** |
| 1 | Mean: 149 nm | Mean: 146 nm | Mean: 295 nm | Mean: 1179 nm |
| | 90% < 289 nm | 90% < 280 nm | 90% < 386 nm | 90% < 2744 nm |
| 2 | Mean: 824 nm | Mean: 927 nm | Mean: 973 nm | Mean: 1099 nm |
| | 90% < 1357 nm | 90% < 1476 nm | 90% < 1526 nm | 90% < 1681 nm |

Stability results indicate that Formulation 1 is preferred over Formulation 2, as Formulation 2 exhibited slight agglomeration in simulated intestinal fluid and unacceptable particle size growth over time.

### Example 2

The purpose of this example was to prepare nanoparticulate dispersions of fenofibrate, followed by testing the stability of the compositions in various simulated biological fluids.

Four formulations of fenofibrate were prepared, as described in Table 4, by milling the components of the compositions in a DYNO^{®}-Mill KDL (Willy A. Bachofen AG, Maschinenfabrik, Basle, Switzerland) for ninety minutes.

Formulation 3 comprised 5% (w/w) fenofibrate, 1% (w/w) hydroxypropylcellulose SL (HPC-SL), and 0.01% (w/w) DOSS; Formulation 4 comprised 5% (w/w) fenofibrate, 1% (w/w) hypromellose, and 0.01% (w/w) DOSS; Formulation 5 comprised 5% (w/w) fenofibrate, 1% (w/w) polyvinylpyrrolidone (PVP K29/32), and 0.01% (w/w) DOSS; and Formulation 6 comprised 5% (w/w) fenofibrate, 1% (w/w) Pluronic^{®} S-630, and 0.01% (w/w) DOSS.

The particle size of the resultant compositions was measured using a Horiba LA-910 Laser Scattering Particle Size Distribution Analyzer ((Horiba Instruments, Irvine, CA).

| **TABLE 4** | | | |
|---|---|---|---|
| **Particle Size of Nanoparticulate** | | | |
| **Fenofibrate Formulations (Comparative Examples)** | | | |
| **Formulation** | **Drug** | **Surface Stabilizer** | **Particle Size** |
| 3 | 5% (w/w) | 1% HPC-SL and 0.01% DOSS | Mean: 696 nm |
| | | | 90% < 2086 nm |
| 4 | 5 % (w/w) | 1% hypromellose and 0.01% DOSS | Mean: 412 nm |
| | | | 90% < 502 nm |
| 5 | 5% (w/w) | 1% PVP and 0.01% DOSS | Mean: 4120 nm |
| | | | 90% < 9162 nm |
| 6 | 5% (w/w) | 1% S630 and 0.01% DOSS | Mean: 750 nm |
| | | | 90% < 2184 nm |

The results indicate that PVP is not a satisfactory surface stabilizer for fenofibrate, at the particular concentrations of fenofibrate and PVP disclosed, in combination with DOSS, as the mean particle size of Formulation 5 was over two microns. However, PVP may be useful as a surface stabilizer for fenofibrate when it is used alone, in combination with another surface stabilizer, or when different concentrations of PVP and/or fenofibrate are utilized.

Next, the stability of Formulations 4 and 6 was tested in various simulated biological fluids (Table 5).

| **TABLE 5** | | | |
|---|---|---|---|
| **Stability Testing of Nanoparticulate Fenofibrate** | | | |
| **Formulations 3-6 in Simulated Biological Fluids** | | | |
| **Formulation** | **Electrolyte Test Media #1** | **Electrolyte Test Media #2** | **Electrolyte Test Media #3** |
| 3 | N/A | N/A | N/A |
| 4 | Acceptable | Acceptable | Acceptable |
| 5 | N/A | N/A | N/A |
| 6 | Agglomeration | Very slight agglomeration | Slight agglomeration |

The results indicate that Formulation 4, comprising hypromellose and DOSS as surface stabilizers, is preferred as the initial particle size is within the useable range (*i.e.,* 90% < 512 nm) and the composition shows no aggregation in various simulated biological fluids.

The next set of examples relate to the redispersibility of the spray granulated powders of the nanoparticulate fenofibrate compositions. The purpose for establishing redispersibility of the spray granulated powder is to determine whether the solid nanoparticulate fenofibrate composition of the invention will redisperse when introduced into *in vitro* or *in vivo* biologically relevant media.

### Example 3

The purpose of this example was to evaluate the redispersibility of spray granulated powders of preferred nanoparticulate fenofibrate compositions comprising hypromellose and DOSS with or without SLS, a preferred small anionic surfactant.

The redispersibility of two powder forms of a spray granulated powder of nanoparticulate fenofibrate was determined, the results of which are shown in Table 6.

**TABLE 6**

| **Physical form** | | | **Powder (Comparative Example)** | **Powder** |
|---|---|---|---|---|
| **Drug:Sucrose** | | | 1:0.6 | 1:1 |
| **Hypromellose:DOSS** | | | 1:0.2 | - |
| **Hypromellose:DOSS +SLS** | | | - | 1:0.3 |
| **Redispersibility** | | | | |
| | **DI water** | | | |
| | | **Mean (nm)** | 390 | 182 |
| | | **D90 (nm)** | 418 | 260 |
| | | **% < 1000 nm** | 95.9 | 100.0 |
| **Electrolyte Test Media #2** | | | | |
| | | **Mean (nm)** | 258 | 193 |
| | | **D90 (nm)** | 374 | 276 |
| | | **% < 1000 nm** | 99.7 | 100.0 |
| **Electrolyte Test Media #3** | | | | |
| | | **Mean (nm)** | 287 | 225 |
| | | **D90 (nm)** | 430 | 315 |
| | | **% < 1000 nm** | 99.6 | 100.0 |

The results show that powders prepared from a granulation feed dispersion having hypromellose, DOSS and SLS exhibit excellent redispersiblity.

### Example 4

The purpose of this example was to test the redispersibility of a spray granulated powder of nanoparticulate fenofibrate comprising higher levels of DOSS and SLS, as compared to Example 3. The results are shown in Table 7.

**TABLE 7**

| **Physical form** | | | **Powder** |
|---|---|---|---|
| **Drug:Sucrose** | | | 1:1 |
| **Hypromellose:SLS + DOSS** | | | 1:0.45 |
| **Redispersibility** | | | |
| | **DI water** | | |
| | | **Mean (nm)** | 196 |
| | | **D90 (nm)** | 280 |
| | | **% < 1000 nm** | 100 |
| **Electrolvte Test Media #2** | | | |
| | | **Mean (nm)** | 222 |
| | | **D90 (nm)** | 306 |
| | | **% < 1000 nm** | 100 |
| **Electrolyte Test Media #3** | | | |
| | | **Mean (nm)** | 258 |
| | | **D90 (nm)** | 362 |
| | | **% < 1000 nm** | 100 |

Excellent redispersibility was observed for all of the tested compositions in simulated biological fluids.

### Example 5

The purpose of this example was to prepare a nanoparticulate fenofibrate tablet formulation.

A fenofibrate nanoparticulate dispersion was prepared by combining the materials listed in Table 8, followed by milling the mixture in a Netzsch LMZ2 Media Mill with Grinding Chamber with a flow rate of 1.0 ± 0.2 LPM and an agitator speed of 3000 ± 100 RPM, utilizing Dow PolyMill^{™} 500 micron milling media. The resultant mean particle size of the nanoparticulate fenofibrate dispersion (NCD), as measured by a Horiba LA-910 Laser Scattering Particle Size Distribution Analyzer ((Horiba Instruments, Irvine, CA) was 169 nm.

| **TABLE 8** | |
|---|---|
| **Nanoparticulate Fenofibrate Dispersion** | |
| **(Comparative Example)** | |
| Fenofibrate | 300 g/Kg |
| Hypromellose, USP (Pharmacoat^{®} 603) | 60 g/Kg |
| Docusate Sodium, USP | 0.75 g/Kg |
| Purified Water | 639.25 g/Kg |

Next, a granulated feed dispersion (GFD) was prepared by combining the nanoparticulate fenofibrate dispersion with the additional components specified in Table 9.

| **TABLE 9** | |
|---|---|
| **Nanoparticulate Fenofibrate Granular Feed Dispersion (Comparative Example)** | |
| Nanoparticulate Fenofibrate Dispersion | 1833.2 g |
| Sucrose, NF | 550.0 g |
| Sodium Lauryl Sulfate, NF | 38.5 g |
| Docusate Sodium, USP/EP | 9.6 g |
| Purified Water | 723.2 g |

The fenofibrate GFD was sprayed onto lactose monohydrate (500 g) to form a spray granulated intermediate (SGI) using a Vector Multi-1 Fluid Bed System set to run at the parameters specified in Table 10, below.

| **TABLE 10** | |
|---|---|
| **Fluid Bed System Parameters** | |
| Inlet Air Temperature | 70 + 10° C |
| Exhaust/Product Air Temperature | 37 ± 5° C |
| Air Volume | 30 + 20 CFM |
| Spray Rate | 15 + 10 g/min |

The resultant spray granulated intermediate (SGI) of the nanoparticulate fenofibrate is detailed in Table 11, below.

| **TABLE 11** | |
|---|---|
| **Spray Granulated Intermediate of the Nanoparticulate Fenofibrate (Comparative Example)** | |
| Fenofibrate NCD | 1833.2 g |
| Sucrose, NF | 550.0 g |
| Sodium Lauryl Sulfate, NF | 38.5 g |
| Docusate Sodium, USP/EP | 9.6 g |
| Lactose Monohydrate, NF | 500 g |

The nanoparticulate fenofibrate SGI was then tableted using a Kilian tablet press with a 0.700 x 0.300" plain upper and lower caplet shape punches. Each tablet has 160 mg of fenofibrate. The resulting tablet formulation is shown below in Table 12.

| **TABLE 12** | |
|---|---|
| **Nanoparticulate Fenofibrate Tablet Formulation (Comparative Example)** | |
| Nanoparticulate Fenofibrate Spray Granulated Intermediate | 511.0 mg |
| Silicified Microcrystalline Cellulose | 95.0 mg |
| Crospovidone, NF | 83.0 mg |
| Magnesium Stearate, NF | 1. 0 mg |

### Example 6

The purpose of this example was to assess the effect of food on the bioavailability of a nanoparticulate fenofibrate tablet formulation, as prepared in Example 5.

### Study Design

A single dose, three way cross-over design study, with eighteen subjects, was conducted. The three treatments consisted of:

| | |
|---|---|
| Treatment A: | 160 mg nanoparticulate fenofibrate tablet administered under fasted conditions; |
| Treatment B: | 160 mg nanoparticulate fenofibrate tablet administered under high fat fed conditions; and |
| Treatment C: | 200 mg micronized fenofibrate capsule (TRICOR^{®}) administered under low fat fed conditions. |

"Low fat fed" conditions are defined as 30% fat - 400 Kcal, and "high fat fed" conditions are defined as 50% fat - 1000 Kcal. The length of time between doses in the study was 10 days.

### Results

Figure 1 shows the plasma fenofibric acid profiles (*i.e.,* the fenofibric acid concentration (µg/ml)) over a period of 120 hours for Treatment A, Treatment B, and Treatment C. Figure 2 shows the same fenofibric acid profiles, but over a 24 hour period rather than a 120 hour period.

Surprisingly, all three Treatments produce approximately the same profile, although the nanoparticulate fenofibrate tablet administered under fasting conditions exhibited a marginally higher maximum fenofibrate concentration. These results are significant for several reasons. First, the nanoparticulate fenofibrate tablet is effective at a lower dosage than that of the conventional microcrystalline fenofibrate capsule: 160 mg vs. 200 mg. A lower dosage is always seen as beneficial for the patient, as less active agent is administered to the patient.

Second, the results show that the nanoparticulate fenofibrate tablet formulation does not exhibit significant differences in absorption when administered in the fed versus the fasted state. This is significant as it eliminates the need for a patient to ensure that they are taking a dose with or without food. Therefore, the nanoparticulate fenofibrate dosage form will result in increased patient compliance. With poor patient compliance an increase in cardiovascular problems or other conditions for which the fenofibrate is being prescribed can result.

The pharmacokinetic parameters of the three tests are shown below in Table 13.

| **TABLE 13** | | | |
|---|---|---|---|
| **Pharmacokinetic Parameters** | | | |
| **(Mean, Standard Deviation, CV%)** | | | |
| | **Treatment A** | **Treatment B** | **Treatment C** |
| AUC (µg/mL.h) | mean = 139.41 | mean = 138.55 | mean = 142.96 |
| | SD = 45.04 | SD = 41.53 | SD = 51.28 |
| | CV% = 32% | CV% = 30% | CV% = 36% |
| Cₘₐₓ (µg/mL) | mean = 8.30 | mean = 7.88 | mean = 7.08 |
| | SD = 1.37 | SD = 1.74 | SD = 1.72 |
| | CV% = 17% | CV% = 22% | CV% = 24% |

The pharmacokinetic parameters first demonstrate that there is no difference in the amount of drug absorbed when the nanoparticulate fenofibrate tablet is administered in the fed versus the fasted condition (*see* the AUC results; 139.41 µg/mL.h for the dosage form administered under fasted conditions and 138.55 µg/mL.h for the dosage form administered under fed conditions). Second, the data show that there was no difference in the *rate* of drug absorption when the nanoparticulate fenofibrate tablet is administered in the fed versus the fasted condition (*see* the Cₘₐₓ results; 8.30 µg/mL for the dosage form administered under fasted conditions and 7.88 µg/mL for the dosage form administered under fed conditions). Thus, the nanoparticulate fenofibrate dosage form eliminates the effect of food on the pharmacokinetics of fenofibrate. Accordingly, the invention encompasses a fibrate composition wherein the pharmacokinetic profile of the fibrate is not affected by the fed or fasted state of a subject ingesting the composition.

### Bioequivalence of the Nanoparticulate Fenofibrate Dosage Form When Administered in the Fed vs Fasted State

Using the data from Table 13, it was determined whether administration of a nanoparticulate fenofibrate tablet in a fasted state was bioequivalent to administration of a nanoparticulate fenofibrate tablet in a fed state, pursuant to regulatory guidelines. The relevant date from Table 13 is shown below in Table 14, along with the 90% Confidence Intervals (CI). Under U.S. FDA guidelines, two products or methods are bioequivalent if the 90% CI for AUC and Cₘₐₓ are between 0.80 to 1.25. As shown below in Table 14, the 90% CI ratio for the nanoparticulate fenofibrate fed/fasted methods is 0.952 : 1.043 for AUC and 0.858 : 1.031 for Cₘₐₓ.

| **TABLE 14** | | | |
|---|---|---|---|
| **Bioequivalence of Nanoparticulate Fenofibrate Tablet HFF vs. Nanoparticulate Fenofibrate Tablet Fasted** | | | |
| | | | **CI 90% on log-transformed data** |
| **AUC (µg/mL.h)** | Nanoparticulate Fenofibrate | 139 | 0.952 : 1.043 |
| | Tablet 160 mg HFF | | |
| | Nanoparticulate Fenofibrate | 139 | |
| | Tablet 160 mg Fasted | | |
| | | | |
| **Cmax (µg/mL)** | Nanoparticulate Fenofibrate | 7.88 | 0.858 : 1.031 |
| | Tablet 160 mg HFF | | |
| | Nanoparticulate Fenofibrate | 8.30 | |
| | Tablet 160 mg Fasted | | |

Accordingly, pursuant to regulatory guidelines, administration of a nanoparticulate fenofibrate tablet in a fasted state is bioequivalent to administration of a nanoparticulate fenofibrate tablet in a fed state. Thus, the invention encompasses a fibrate composition wherein administration of the composition to a subject in a fasted state is bioequivalent to administration of the composition to a subject in a fed state.

Moreover, as shown by the data in Table 15 below, administration of a 160 mg nanoparticulate fenofibrate tablet in a fed state is bioequivalent to administration of a 200 mg conventional microcrystalline fenofibrate capsule (TRICOR^{®}) in a fed state. This is because CI 90% for the two treatments is within 0.80 to 1.25 for AUC and Cₘₐₓ.

| **TABLE 15** | | | |
|---|---|---|---|
| **Bioequivalence of Nanoparticulate 160 mg Fenofibrate Tablet HFF vs. a Microcrystalline 200 mg Fenofibrate Capsule (TRICOR^{®}) HFF** | | | |
| | | | **CI 90% on log-transformed data** |
| **AUC (µg/mL.h)** | Nanoparticulate 160 mg Fenofibrate Tablet HFF | 139 | 0.936 : 1.026 |
| | Microcrystalline 200 mg | 143 | |
| | Fenofibrate Capsule | | |
| | (TRICOR^{®}) HFF | | |
| | | | |
| **Cmax (µg/mL)** | Nanoparticulate 160 mg | 7.88 | 1.020 : 1.226 |
| | Fenofibrate Tablet HFF | | |
| | Microcrystalline 200 mg | 7.08 | |
| | Fenofibrate Capsule | | |
| | (TRICOR^{®}) HFF | | |

Finally, as shown by the data in Table 16, below, administration of a 160 mg nanoparticulate fenofibrate tablet in a fasted state is ***not*** bioequivalent to administration of a 200 mg conventional microcrystalline fenofibrate capsule (TRICOR^{®}) in a fed state. This is because CI 90% for the two treatments is ***outside*** 0.80 to 1.25 for AUC and Cₘₐₓ.

| **TABLE 16** | | | |
|---|---|---|---|
| **Non-Bioequivalence of Nanoparticulate 160 mg Fenofibrate Tablet Fasted vs. a Microcrystalline 200 mg Fenofibrate Capsule (TRICOR^{®}) HFF** | | | |
| | | | **CI 90% on log-transformed data** |
| **AUC (µg/mL.h)** | Nanoparticulate 160 mg | 139 | 0.939 : 1.030 |
| | Fenofibrate Tablet Fasted | | |
| | Microcrystalline 200 mg | 143 | |
| | Fenofibrate Capsule | | |
| | (TRICOR^{®}) HFF | | |
| | | | |
| **Cmax (µg/mL)** | Nanoparticulate 160 mg | 8.30 | 1.084 : 1.304 |
| | Fenofibrate Tablet Fasted | | |
| | Microcrystalline 200 mg | 7.08 | |
| | Fenofibrate Capsule | | |
| | (TRICOR^{®}) HFF | | |

The non-bioequivalence is significant, because it means that the nanoparticulate fenofibrate dosage form exhibits significantly greater drug absorption. For the nanoparticulate fenofibrate dosage form to be bioequivalent to the conventional microcrystalline fenofibrate dosage form (e.g., TRICOR^{®}), the dosage form would have to contain significantly less drug. Thus, the nanoparticulate fenofibrate dosage form significantly increases the bioavailability of the drug.

### Example 7

The purpose of this example was to provide nanoparticulate fenofibrate tablet formulations prepared as described in Example 5, above.

Shown below in Table 17 is the nanoparticulate fenofibrate dispersion used for making the nanoparticulate fenofibrate tablet formulations.

| **TABLE 17** | |
|---|---|
| **Nanoparticulate Fenofibrate Dispersion** | |
| Fenofibrate | 194.0 g/Kg |
| Hypromellose, USP (Pharmacoat^{®} 603) | 38.81 g/Kg |
| Docusate Sodium, USP | 0.485 g/Kg |
| Water for injection, USP, EP | 572.7 g/Kg |
| Sucrose, NF | 194.0 g/Kg |
| **Actual Total** | 1000.0 |

Two different tablets were made using the dispersion: a 145 mg nanoparticulate fenofibrate tablet and a 48 mg nanoparticulate fenofibrate table.

A granulated feed dispersion (GFD) was prepared by combining the nanoparticulate fenofibrate dispersion with sucrose, docusate sodium, and sodium lauryl sulfate.

The fenofibrate GFD was processed and dried in a fluid-bed column (Vector Multi-1 Fluid Bed System), along with lactose monohydrate. The resultant spray granulated intermediate (SGI) was processed through a cone mill, followed by (1) processing in a bin blender with silicified microcrystalline cellulose and crospovidone, and (2) processing in a bin blender with magnesium stearate. The resultant powder was tableted in a rotary tablet press, followed by coating with Opadry^{®} AMB using a pan coater.

Table 18 provides the composition of the 145 mg fenofibrate tablet, and Table 19 provides the composition of the 48 mg fenofibrate tablet.

| **TABLE 18** | |
|---|---|
| **145 mg Nanoparticulate** | |
| **Fenofibrate Tablet Formulation** | |
| **Component** | **g/Kg** |
| Fenofibrate | 222.54 |
| Hypromellose, USP | 44.506 |
| Docusate Sodium, USP | 4.4378 |
| Sucrose, NF | 222.54 |
| Sodium Lauryl Sulfate, NF | 15.585 |
| Lactose Monohydrate, NF | 202.62 |
| Silicified Microcrystalline Cellulose | 132.03 |
| Crospovidone, NF | 115.89 |
| Magnesium Stearate, NF | 1.3936 |
| Opadry OY-28920 | 38.462 |
| **Actual Total** | 1000.0 |

| **TABLE 19** | |
|---|---|
| **48 mg Nanoparticulate** | |
| **Fenofibrate Tablet Formulation** | |
| **Component** | **g/Kg** |
| Fenofibrate | 221.05 |
| Hypromellose, USP | 44.209 |
| Docusate Sodium, USP | 4.4082 |
| Sucrose, NF | 221.05 |
| Sodium Lauryl Sulfate, NF | 15.481 |
| Lactose Monohydrate, NF | 201.27 |
| Silicified Microcrystalline Cellulose | 131.14 |
| Crospovidone, NF | 115.12 |
| Magnesium Stearate, NF | 1.3843 |
| Opadry OY-28920 | 44.890 |
| **Actual Total** | 1000.0 |

### Example 8

The purpose of this example was to compare the dissolution of a nanoparticulate 145 mg fenofibrate dosage form according to the invention with a conventional microcrystalline form of fenofibrate (TRICOR^{®}) in a dissolution medium which is representative of *in vivo* conditions.

The dissolution of the 145 mg nanoparticulate fenofibrate tablet, prepared in Example 7, was tested in a dissolution medium which is discriminating. Such a dissolution medium will produce two very different dissolution curves for two products having very different dissolution profiles in gastric juices; *i.e.,* the dissolution medium is predictive of *in vivo* dissolution of a composition.

The dissolution medium employed was an aqueous medium containing the surfactant sodium lauryl sulfate at 0.025 M. Determination of the amount dissolved was carried out by spectrophotometry, and the tests were repeated 12 times. The rotating blade method (European Pharmacopoeia) was used under the following conditions:
volume of media: 1000 ml;
media temperature: 37 °C;
blade rotation speed: 75 RPM;
samples taken: every 2.5 minutes;

The results are shown below in Table 20. The table shows the amount (%) of the solid dosage form dissolved at 5, 10, 20, and 30 minutes for twelve different samples, as well as the mean (%) and standard deviation (%) results.

| **TABLE 20** | | | | |
|---|---|---|---|---|
| **Dissolution Profile of the Nanoparticulate Fenofibrate 145 mg Table** | | | | |
| **Test Sample** | **5 min.** | **10 min.** | **20 min.** | **30 min.** |
| 1 | 36.1 | 80.9 | 101.7 | 103.6 |
| 2 | 73.4 | 100.5 | 100.1 | 101.8 |
| 3 | 44.0 | 85.6 | 100.0 | 101.4 |
| 4 | 41.0 | 96.1 | 102.3 | 102.5 |
| 5 | 58.7 | 92.9 | 103.4 | 103.5 |
| 6 | 51.9 | 97.8 | 102.6 | 103.4 |
| 7 | 28.6 | 66.9 | 99.3 | 100.4 |
| 8 | 44.7 | 97.4 | 98.8 | 99.3 |
| 9 | 30.1 | 76.9 | 97.0 | 98.0 |
| 10 | 33.6 | 76.8 | 101.8 | 103.5 |
| 11 | 23.5 | 52.6 | 95.8 | 104.0 |
| 12 | 34.6 | 66.9 | 102.8 | 102.2 |
| Mean (%) | 41.7 | 82.6 | 100.5 | 102.0 |
| Standard Deviation (%) | 14.1 | 15.2 | 2.4 | 1.9 |

U.S. Patent No. 6,277,405, for "Fenofibrate Pharmaceutical Composition Having High Bioavailability and Method for Preparing It," describes dissolution of a conventional microcrystalline 160 mg fenofibrate dosage form, *e.g.,* TRICOR^{®}, using the same method described above for the nanoparticulate fenofibrate dosage form (Example 2, cols. 8-9). The results show that the conventional fenofibrate dosage form has a dissolution profile of 10% in 5 min., 20% in 10 min., 50% in 20 min., and 75% in 30 min.

The results show that the nanoparticulate fenofibrate dosage form had dramatically more rapid dissolution as compared to the conventional microcrystalline form of fenofibrate. For example, while within 5 minutes approximately 41.7% of the nanoparticulate fenofibrate dosage form had dissolved, only 10% of the TRICOR^{®} dosage form had dissolved. Similarly, while at 10 min. about 82.6% of the nanoparticulate fenofibrate dosage form was dissolved, only about 20% of the TRICOR^{®} dosage form had dissolved during the same time period. Finally, while at 30 min. basically 100% of the nanoparticulate dosage form had dissolved, only about 75% of the conventional fenofibrate dosage form had dissolved during the same time period.

Thus, the nanoparticulate fenofibrate dosage forms of the invention exhibit dramatically improved rates of dissolution.

The invention is defined by the appended claims.

## Claims

1. A fenofibrate composition for oral administration comprising:
(a) particles of fenofibrate or a salt thereof,
wherein at least 99% of the fibrate particles by weight have a particle size of less than 500nm or at least 50% of the fibrate particles by weight have a particle size of less than 350nm; and
(b) associated with the surface thereof, a surface stabilizer composition comprising hypromellose, docusate sodium, and sodium lauryl sulfate,
wherein the composition does not comprise PEG-derivatized vitamin E, and
wherein the composition does not produce significantly different absorption levels when administered underfed as compared to fasting conditions
wherein administration of the composition to a subject in a fasted state is bioequivalent to administration of the composition to a subject in a fed state, wherein "bioequivalency" is established by:
(a) a 90% Confidence Interval (CI) of between 0.80 and 1.25 for both Cmax and AUC under USFDA regulatory guidelines or
(b) a 90% Confidence Interval (CI) for AUC of between 0.80 and 1.25 and a 90% Cl for Cmax of between 0.70 to 1.43 under the European EMEA regulatory guidelines.

2. The composition of claim 1, wherein the fenofibrate is selected from the group consisting of a crystalline phase, an amorphous phase, a semi-crystalline phase, a semi-amorphous phase, and mixtures thereof.

3. The composition of claim 1 or 2, wherein at least 50% of the fenofibrate particles, by weight, have a particle size selected from the group consisting of less than 300 nm, less than 250 nm, less than 200 nm, less than 100 nm, less than 75 nm, and less than 50 nm.

4. The composition of any one of claims 1-3 wherein the composition further comprises one or more pharmaceutically acceptable excipients, carriers, or a combination thereof.

5. The composition of any one of claims 1-4, wherein the fibrate is present in an amount selected from the group consisting of from 99.5% to 0.001%, from 95% to 0.1%, and from 90% to 0.5%, by weight, based on the total combined weight of the fibrate and at least the three surface stabilizers, not including other excipients.

6. The composition of any one of claims 1-5, wherein the at least three surface stabilizers are present in an amount selected from the group consisting of from 0.5% to 99.999% by weight, from 5.0% to 99.9% by weight, and from 10% to 99.5% by weight, based on the total combined dry weight of the fibrate and at least one surface stabilizer, not including other excipients.

7. The composition of any one of claims 1-6, wherein the composition exhibits a Tmax selected from the group consisting of less than 6 hours, less than 5 hours, less than 4 hours, less than 3 hours, less than 2 hours, less than 1 hour, and less than 30 minutes after administration to fasting subjects.

8. The composition of claim 2, wherein the difference in absorption of the fibrate composition of the invention, when administered in the fed versus the fasted state, is selected from the group consisting of less than 35%, less than 30%, less than 25%, less than 20%, less than 15%, less than 10%, less than 5%, and less than 3%.

9. The composition of any one of claims 1-8, comprising fenofibrate or a salt thereof, wherein in comparative pharmacokinetic testing with a TRICOR^{®} 160 mg tablet or 200 mg capsule, which are standard commercial formulations of microcrystalline fenofibrate, the composition exhibits a Tmax, selected from the group consisting of less than 90%, less than 80%, less than 70%, less than 50%, less than 30%, and less than 25% of the Tmax exhibited by the TRICOR^{®} tablet or capsule.

10. The composition of any one of claims 1-9, wherein the fibrate particles have a particle size in which the D₅₀ is less than 350 nm.

11. The composition of any one of claims 1-10, for use in the treatment of a condition selected from the group consisting of hypercholesterolemia, hypertriglyceridemia, coronary heart disease, cardiovascular disorders, and peripheral vascular disease.

12. The composition of claim 1, further comprising a HMG-CoA reductase inhibitor.

13. The composition of claim 12, wherein the HMG-CoA reductase inhibitor is selected from the group consisting of lovastatin; pravastatin; simvastatin; velostatin; atorvastatin; fluvastatin; fluindostatin; rivastatin; Searle's SC-45355; dichloroacetate;; octahydronaphthalenes; and phosphinic acid compounds.

14. A solid dosage form comprising a composition as claimed in any of the preceding claims.

15. The dosage form of claim 14 which is in the form of a tablet.

16. The dosage form of claim 14 or 15, wherein the fenofibrate or a salt thereof is admixed with at least one of the following:
(a) one or more inert excipients or carriers,
(b) fillers or extenders,
(c) binders,
(d) humectants,
(e) disintegrating agents,
(f) solution retarders,
(g) absorption accelerators,
(h) wetting agents,
(i) adsorbents, and
(j) lubricants.

17. The dosage form of claim 14, 15 or 16, further comprising a HMG-CoA reductase inhibitor.

18. The dosage form of claim 17, wherein the HMG-CoA reductase inhibitor is selected from the group consisting of lovastatin; pravastatin; simvastatin; velostatin; atorvastatin; fluvastatin; fluindostatin; rivastatin; Searle's SC-45355; dichloroacetate; octahydronaphthalenes; and phosphinic acid compounds.

## Patentansprüche

1. Fenofibratzusammensetzung zur oralen Verabreichung, diese umfasst:
(a) Partikel von Fenofibrat oder einem Salz davon,
wobei mindestens 99 Gew.-% der Fibratpartikel eine Partikelgröße von weniger als 500 nm aufweisen oder mindestens 50 Gew.-% der Fibratpartikel eine Partikelgröße von weniger als 350 nm aufweisen, und
(b) mit der Oberfläche derselben, eine Oberflächenstabilisatorzusammensetzung verbunden ist, die Hypromellose, Dokusat-Natrium und Natriumlaurylsulfat umfasst,
wobei die Zusammensetzung kein PEG-derivatisiertes Vitamin E umfasst, und
wobei die Zusammensetzung, im Vergleich zu nüchternen Bedingungen, keine signifikant unterschiedlichen Absorptionsniveaus erzeugt, wenn sie unter Nahrungszufuhr verabreicht wird,
wobei die Verabreichung der Zusammensetzung an einen Probanden in einem nüchternen Zustand bioäquivalent zur Verabreichung der Zusammensetzung an einen Probanden in einem ernährten Zustand ist, wobei die "Bioäquivalenz" durch Folgendes bestimmt wird:
(a) ein Konfidenzintervall (Kl) von 90 % zwischen 0,80 und 1,25 sowohl für Cmax als auch AUC gemäß den USFDA-Richtlinien oder
(b) ein Konfidenzintervall (KI) von 90 % für AUC zwischen 0,80 und 1,25 und ein KI von 90 % für Cmax zwischen 0,70 und 1,43 gemäß den europäischen EMEA-Richtlinien.

2. Zusammensetzung des Anspruchs 1, wobei das Fenofibrat aus einer Gruppe ausgewählt ist, die aus einer kristallinen Phase, einer amorphen Phase, einer halbkristallinen Phase, einer halbamorphen Phase und Mischungen davon besteht.

3. Die Zusammensetzung des Anspruchs 1 oder 2, wobei mindestens 50 Gew.-% der Fenofibratpartikel eine Partikelgröße aufweisen, die aus der Gruppe bestehend aus weniger als 300 nm, weniger als 250 nm, weniger als 200 nm, weniger als 100 nm, weniger als 75 nm und weniger als 50 nm ausgewählt wurde.

4. Die Zusammensetzung der Ansprüche 1 bis 3, wobei die Zusammensetzung ferner einen oder mehrere pharmazeutisch akzeptable Hilfsstoffe, Träger oder eine Kombination dieser umfasst.

5. Die Zusammensetzung der Ansprüche 1 bis 4, wobei das Fibrat in einer Menge vorhanden ist, die aus einer Gruppe bestehend aus 99,5 Gew.-% bis 0,001 Gew.-%, von 95 Gew.-% bis 0,1 Gew.-% und von 90 Gew.-% bis 0,5 Gew.-%, basierend auf dem kombinierten Gesamtgewicht des Fibrats und mindestens den drei Oberflächenstabilisatoren, ohne andere Hilfsstoffe ausgewählt wurde.

6. Die Zusammensetzung der Ansprüche 1 bis 5, wobei die mindestens drei Oberflächenstabilisatoren in einer Menge vorhanden sind, die aus einer Gruppe bestehend aus 0,5 Gew.-% bis 99,999 Gew.-%, von 5,0 Gew.-% bis 99,9 Gew.-% und von 10 Gew.-% bis 99,5 Gew.-%, basierend auf dem kombinierten Gesamttrockengewicht des Fibrats und mindestens einem Oberflächenstabilisator, ohne andere Hilfsstoffe ausgewählt wurde.

7. Zusammensetzung der Ansprüche 1 bis 6, wobei die Zusammensetzung eine Tmax aufweist, die aus einer Gruppe bestehend aus weniger als 6 Stunden, weniger als 5 Stunden, weniger als 4 Stunden, weniger als 3 Stunden, weniger als 2 Stunden, weniger als 1 Stunde und weniger als 30 Minuten nach Verabreichung an nüchterne Probanden ausgewählt wurde.

8. Zusammensetzung des Anspruchs 2, wobei der Unterschied in der Absorption der Fibratzusammensetzung der Erfindung, wenn sie im ernährten verglichen mit dem nüchternen Zustand verabreicht wird, aus einer Gruppe bestehend aus weniger als 35 %, weniger als 30 %, weniger als 25 %, weniger als 20 %, weniger als 15 %, weniger als 10 %, weniger als 5 % und weniger als 3 % ausgewählt wurde.

9. Zusammensetzung der Ansprüche 1 bis 8, die Fenofibrat oder ein Salz davon enthalten, wobei die Zusammensetzung in vergleichenden pharmakokinetischen Tests mit einer TRICOR^{®} 160 mg Tablette oder 200 mg Kapsel, die handelsübliche Standardformulierungen von mikrokristallinem Fenofibrat sind, eine Tmax aufweist, die aus einer Gruppe bestehend aus weniger als 90 %, weniger als 80 %, weniger als 70 %, weniger als 50 %, weniger als 30 % und weniger als 25 % der Tmax, die durch die TRICOR^{®} Tablette oder Kapsel gezeigt wird ausgewählt wurde.

10. Zusammensetzung der Ansprüche 1 bis 9, wobei die Fibratpartikel eine Partikelgröße aufweisen, in der der Dso kleiner als 350 nm ist.

11. Zusammensetzung der Ansprüche 1 bis 10 zur Verwendung bei der Behandlung einer Erkrankung, die aus einer Gruppe bestehend aus Hypercholesterinämie, Hypertriglyzeridämie, koronarer Herzkrankheit, kardiovaskulären Störungen und peripherer Gefäßerkrankung ausgewählt wurde.

12. Zusammensetzung des Anspruchs 1, die zusätzlich einen HMG-CoA-Reduktasehemmer enthält.

13. Zusammensetzung des Anspruchs 12, bei der der HMG-CoA-Reduktaseinhibitor aus einer Gruppe bestehend aus Lovastatin; Pravastatin; Simvastatin; Velostatin; Atorvastatin; Fluvastatin; Fluindostatin; Rivastatin; Searle's SC-45355; Dichloracetat; Octahydronaphthalinen und Phosphinsäureverbindungen ausgewählt wurde.

14. Feste Darreichungsform, die eine Zusammensetzung nach einem der vorhergehenden Ansprüche enthält.

15. Darreichungsform des Anspruchs 14, die in Form einer Tablette vorliegt.

16. Darreichungsform des Anspruchs 14 oder 15, wobei das Fenofibrat oder ein Salz davon mindestens einem der folgenden Stoffe beigemischt wird:
(a) einem oder mehreren inerten Hilfsstoffen oder Trägern,
(b) Füllern oder Verlängerungen,
(c) Bindemitteln,
(d) Feuchthaltemitteln,
(e) Zersetzungsmitteln,
(f) Lösungsverzögerern,
(g) Absorptionsbeschleunigern,
(h) Benetzungsmitteln,
(i) Adsorbenzien und
(j) Schmierstoffen.

17. Darreichungsform des Anspruchs 14, 15 oder 16, die zusätzlich einen HMG-CoA-Reduktasehemmer umfasst.

18. Darreichungsform des Anspruchs 17, wobei der HMG-CoA-Reduktasehemmer aus einer Gruppe bestehend aus Lovastatin; Pravastatin; Simvastatin; Velostatin; Atorvastatin; Fluvastatin; Fluindostatin; Rivastatin; Searle's SC-45355; Dichloroacetat; Octahydronaphthalinen und Phosphinsäureverbindungen ausgewählt wurde.

## Revendications

1. Composition de fénofibrate pour administration orale comprenant :
(a) particules de fénofibrate ou un sel de ce dernier.
dans lequel au moins 99 % des particules de fibrate en poids ont une taille de particule inférieure à 500 nm ou au moins 50 % des particules de fibrate en poids ont une taille de particule inférieure à 350 nm ; et
(b) associée à la surface de celle-ci, une composition stabilisante de surface comprenant de l'hypromellose, du docusate de sodium et du laurylsulfate de sodium,
dans lequel la composition ne comprend pas de vitamine E dérivée du PEG, et
dans lequel la composition ne produit pas de niveaux d'absorption significativement différents lorsqu'il est administré dans des conditions postprandiales par rapport à des conditions de jeûne
dans lequel l'administration de la composition à un sujet à jeun est bioéquivalente à l'administration de la composition à un sujet à l'état postprandial, dans lequel la « bioéquivalence » est établie par :
(a) un intervalle de confiance (Cl) à 90 % compris entre 0,80 et 1,25 pour la Cmax et l'AUC selon les directives réglementaires de la FDA américaine ou
(b) un intervalle de confiance (IC) à 90 % pour une AUC comprise entre 0,80 et 1,25 et une IC à 90 % pour une Cmax comprise entre 0,70 et 1,43 selon les directives réglementaires européennes de la région EMEA.

2. Composition selon l'affirmation 1, dans laquelle le fénofibrate est choisi dans le groupe constitué d'une phase cristalline, d'une phase amorphe, d'une phase semi-cristalline, d'une phase semi-amorphe et de leurs mélanges.

3. Composition selon l'affirmation 1 ou 2, dans laquelle au moins 50 % des particules de fénofibrate, en poids, ont une taille de particule choisie dans le groupe constitué de moins de 300 nm, moins de 250 nm, moins de 200 nm, moins de 100 nm, moins de 75 nm et moins de 50 nm.

4. Composition selon l'une des affirmations 1 à 3, dans laquelle la composition comprend en outre un ou plusieurs excipients ou supports pharmaceutiquement acceptables, ou une combinaison de ces derniers.

5. Composition selon l'une des affirmations 1 à 4, dans laquelle le fibrate est présent dans une quantité sélectionnée dans le groupe constitué de 99,5 % à 0,001 %, de 95 % à 0,1 %, et de 90 % à 0,5 %, en poids, sur la base du poids total combiné du fibrate et au moins des trois stabilisateurs de surface, sans inclure d'autres excipients.

6. Composition selon l'une des affirmations 1 à 5, dans laquelle au moins trois des stabilisateurs de surface sont présents dans une quantité sélectionnée dans le groupe constitué de 0,5 % à 99,999 % en poids, de 5,0 % à 99,9 % en poids, et de 10 % à 99,5 % en poids, sur la base du poids total combiné à sec du fibrate et d'au moins un stabilisateur de surface, sans inclure d'autres excipients.

7. Composition selon l'une des affirmations 1 à 6, dans laquelle la composition présente un Tmax choisi dans le groupe constitué de moins de 6 heures, moins de 5 heures, moins de 4 heures, moins de 3 heures, moins de 2 heures, moins de 1 heure et moins de 30 minutes après l'administration à des sujets à jeun.

8. Composition selon l'affirmation 2, dans laquelle la différence d'absorption de la composition de fibrate de l'invention, lorsqu'elle est administrée à l'état postprandial par rapport à l'état à jeun, est choisie dans le groupe constitué de moins de 35 %, moins de 30 %, moins de 25 %, moins de 20 %, moins de 15 %, moins de 10 %, moins de 5 % et moins de 3 %.

9. Composition selon l'une des affirmations 1 à 8, comprenant du fénofibrate ou un sel de celui-ci, dans laquelle, lors d'essais pharmacocinétiques comparatifs avec un comprimé de TRICOR^{®} 160 mg ou une gélule de 200 mg, qui sont des formulations commerciales standard de fénofibrate microcristallin, la composition présente un Tmax, choisi dans le groupe constitué de moins de 90 %, moins de 80 %, moins de 70 %, moins de 50 %, moins de 30 % et moins de 25 % du Tmax présenté par le comprimé ou la gélule de TRICOR^{®}.

10. Composition selon l'une des affirmations 1 à 9, dans laquelle les particules de fibrate ont une taille de particule dans laquelle le Dso est inférieur à 350 nm.

11. Composition selon l'une des affirmations 1 à 10, destinée à être utilisée dans le traitement d'une affection choisie dans le groupe constitué par l'hypercholestérolémie, l'hypertriglycéridémie, la maladie coronarienne, les troubles cardiovasculaires et la maladie vasculaire périphérique.

12. Composition selon l'affirmation 1, comprenant en outre un inhibiteur de la HMG-CoA réductase.

13. Composition selon l'affirmation 12, dans laquelle l'inhibiteur de l'HMG-CoA réductase est choisi parmi
le groupe composé de lovastatine ; pravastatine ; simvastatine ; velostatine ; atorvastatine ; fluvastatine ; fluindostatine ; rivastatine ; SC-45355 de Searle ; dichloroacétate ; octahydronaphtalènes ; et composés d'acide phosphinique.

14. Forme galénique solide comprenant une composition selon l'une des affirmations précédentes.

15. Forme galénique selon l'affirmation 14, qui se présente sous la forme d'un comprimé.

16. Forme galénique selon l'affirmation 14 ou 15, dans laquelle le fénofibrate ou un sel de celui-ci est mélangé
avec au moins l'un des éléments suivants :
(a) un ou plusieurs excipients ou porteurs inertes,
(b) produits de comblement ou des prolongateurs,
(c) liants,
(d) humectants,
(e) agents désintégrants,
(f) ralentisseurs de solution,
(g) accélérateurs d'absorption,
(h) agents humidifiants,
(i) adsorbants, et
(j) lubrifiants.

17. Forme galénique selon l'affirmation 14, 15 ou 16, comprenant en outre un inhibiteur de l'HMG-CoA réductase.

18. Forme galénique selon l'affirmation 17, dans laquelle l'inhibiteur de l'HMG-CoA réductase est choisi dans le groupe composé de lovastatine ; pravastatine ; simvastatine ; velostatine ; atorvastatine ; fluvastatine ; fluindostatine ; rivastatine ; SC-45355 de Searle ; dichloroacétate ; octahydronaphtalènes ; et composés d'acide phosphinique.
